# EUROPÄISCHE PATENTANMELDUNG

(11) **EP 4 036 212 A1**
(43) Veröffentlichungstag der Anmeldung: **03.08.2022**
(21) Anmeldenummer: 21153810.3
(22) Anmeldetag: 27.01.2021
(51) Int. Cl.: C12M 3/06, C12M 1/00, C12M 1/36

(54) **INKUBATOR UND VERFAHREN**

(71) Anmelder: Eppendorf AG, 22339 Hamburg (DE)
(72) Erfinder: JANKOWSKI, Alexandra, 22339 Hamburg (DE); KAYSER, Philipp, 22339 Hamburg (DE); HELLWEG, Wolf Lukas, 22339 Hamburg (DE); LEHRKE, Niklas, 22339 Hamburg (DE)
(74) Vertreter: Kirchner, Christian

(57) **Zusammenfassung**

Die Erfindung bezieht sich auf einen Inkubator für lebende Zellkulturen, ein Verfahren zur Arbeit mit einem Inkubator und ein System mit einem Inkubator. Ein Bilderfassungssystem wird verwendet, um eine Belegung des Inkubators zu ermitteln.

## Beschreibung

Die Erfindung bezieht sich auf einen Inkubator für lebende Zellkulturen, ein Verfahren zur Arbeit mit einem Inkubator und ein System mit einem Inkubator.

Mit solchen Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen CO₂-Inkubatoren. Die Atmosphäre wird durch Luft mit einem bestimmten CO₂- und O₂-Gehalt und einer bestimmten Luftfeuchtigkeit gebildet, eine geeignete Temperatur ist oftmals 37 °C.

Das Wachstum der Zellen hängt insbesondere kritisch von der Konstanz der atmosphärischen Bedingungen im Inkubator ab. Störungen der Inkubatoratmosphäre können sich negativ auf das Wachstum der Zellen auswirken. In einem dahingehend "ideal" ausgerüsteten Labor würde man jedem einzelnen Nutzer für jede zu inkubierende Probe eine separat zugängliche Inkubationskammer zur Verfügung stellen. Dies ist aber aus Gründen der Kosteneffizienz nicht realistisch. In der Praxis der Labore ist es üblich, dass ein einzelner Inkubator (oder wenige Inkubatoren) mit einer einzigen Inkubationskammer und einem oder mehreren Lagerbereichen auf einer oder mehreren Lagerplatten in der Inkubationskammer für die Verwendung durch mehrere Benutzer vorgesehen ist.

Gemäß den dieser Erfindung zugrunde liegenden Untersuchungen skaliert die Häufigkeit des Öffnens der Kammertüre des Inkubators und damit des Eingriffs in die geregelte Atmosphäre des Inkubators mit der Anzahl der Nutzer und zudem mit der Anzahl der dort inkubierten Proben. Die Intensität der Störung hängt zudem von der Dauer der Türöffnung ab. Je mehr Zeit ein Nutzer für den Zugriff auf den Innenraum der Inkubatorkammer benötigt, desto länger bleibt die Tür geöffnet.

Es gibt verschiedene Nutzungsszenarien des Inkubators, die durch Komplikationen eine erhöhte Zugriffszeit erfordern können:

### Szenario A) Einstellen neuer Objekte in den Inkubator

Stellt ein Nutzer einen oder mehrere Objekte, insbesondere Zellkulturbehälter, in den Inkubator ein, benötigt er einen freien Lagerplatz in einem Lagerbereich. Sind wegen ungeordneter Lagerung keine freien Lagerplätze zugänglich, benötigt der Nutzer Zeit um diesen Lagerplatz zu schaffen; je sorgfältiger er dabei im Inkubator bereits vorhandene Objekte (Bestandsobjekte) verschiebt bzw. umräumt, dies eventuell sogar schriftlich dokumentiert, desto zeitaufwändiger wird der Vorgang. Erweist es sich dabei, dass in der Inkubatorkammer kein ausreichender Lagerplatz mehr verfügbar ist, so wiederholt der Nutzer sein Vorgehen bei einem weiteren Kompartiment der Inkubatorkammer oder bei einem eventuell vorhandenen Ersatzinkubator des Labors. Dadurch verlängert sich der Zeitraum des Zustands einer geöffneten Inkubatortüre, also die Dauer der Exposition des Kammerinneren zur Umgebung (Expositionsdauer).

### Szenario B) Prüfen von Zellkulturen

Prüft ein Nutzer die von ihm zuvor in den Inkubator eingestellten Zellkulturen, z.B. um die Qualität des Zellmediums oder den Zustand des Wachstums zu beurteilen, so wird der Nutzer zunächst den betreffenden Zellkulturbehälter im Inkubator suchen. Dies verlängert die Expositionsdauer. Je sorgfältiger der Nutzer dabei Bestandsobjekte verschiebt bzw. umräumt, desto zeitaufwändiger wird der Suchvorgang.

### Szenario C) Entnehmen der Objekte aus dem Inkubator

Auch in diesem Fall muss der Nutzer das entsprechende Objekt erst suchen. Es gelten die in B) genannten zeitverzögernden Faktoren.

Je häufiger zudem ein Inkubator geöffnet wird, desto höher ist das Risiko der Kontamination des Innenraums. Es gibt auch Fälle, beispielsweise in der Forensik oder der Reproduktionsmedizin, in denen der Wert einer einzelnen Probe, insbesondere in einem Zellkulturgefäß befindlichen Zelle/n, viel höher einzuschätzen ist als, beispielsweise, der Wert des gesamten Inkubators, so dass ein kontaminationsbedingter Verlust der Probe unbedingt zu vermeiden ist. In jedem Fall erhöht die Kontaminationshäufigkeit das Risiko von Arbeitsausfall, erhöht die Kosten und fordert zusätzlichen Wartungsaufwand. Nach einer Kontamination eines Inkubators muss die Kammer gereinigt und sterilisiert werden, bevor der Inkubator weiter genutzt werden kann. In dieser Zeit ist, insofern kein Ersatzinkubator verfügbar ist, die Arbeit mit Zellkulturen unterbrochen.

In Laboren besteht daher grundsätzlich das Bedürfnis, den Zeitraum möglichst kurz zu halten, während dem die Inkubatortüre geöffnet ist, und zudem, die Häufigkeit des Öffnens der Inkubatorkammer möglichst gering zu halten.

Die der vorliegenden Erfindung zugrunde liegende Aufgabe ist es, einen effizient nutzbaren Inkubator bereitzustellen und, der es insbesondere ermöglicht, die Expositionsdauer, also den Zeitraum des Zustands einer geöffneten Inkubatortüre, und damit die Dauer der Exposition des Inneren der Inkubatorkammer zur Umgebung des Inkubators gering zu halten.

Die Erfindung löst diese Aufgabe durch den Inkubator gemäß Anspruch 1, das System gemäß Anspruch 16, und das Verfahren gemäß Anspruch 17. Bevorzugte Ausgestaltungen sind insbesondere Gegenstände der Unteransprüche.

Der erfindungsgemäße Inkubator zur Inkubation lebender Zellkulturen, weist auf:
Inkubator zur Inkubation lebender Zellkulturen, aufweisend
eine Inkubatorkammer zur Aufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt,
eine Inkubatortüre zum Verschließen der Kammeröffnung,
ein Bilderfassungssystem, aufweisend
   - eine Beleuchtungseinrichtung,
   - mindestens eine Kameraeinrichtung und
   - eine Datenverarbeitungseinrichtung mit einem Datenspeicher,
      wobei das Bilderfassungssystem dazu eingerichtet ist, insbesondere bei geschlossener oder geöffneter Inkubatortüre,
   - mittels der Beleuchtungseinrichtung den sich zwischen den Innenwänden erstreckenden Lagerbereich zu beleuchten,
   - mittels der Kameraeinrichtung mindestens ein Bild des sich zwischen den Innenwänden erstreckenden Lagerbereich zu erfassen, und
   - das mindestens eine Bild mittels der Datenverarbeitungseinrichtung in Form von Bilddaten in der Datenspeichereinrichtung zu speichern.

Durch das Bilderfassungssystem ist es möglich, in der Inkubatorkammer unter kontrollierten Bedingungen und reproduzierbarer Weise Bildaufnahmen anzufertigen, die entsprechend vielseitig verwendbare Informationen über die Belegung des Lagerbereichs liefern können. Da der erfindungsgemäße Inkubator die Information über die Belegung des Lagerbereichs in Form von Bilddaten bereithält, wird es den Benutzern ermöglicht, Informationen über den Belegungszustand des Inkubators vor dem Öffnen des Inkubators abzurufen. Durch das Bereitstellen dieser Information wird unnötiges Öffnen des Inkubators reduziert und die Benutzung des Inkubators wird effizienter.

Die Aufbereitung dieser Information über die Belegung des Lagerbereichs kann darin bestehen, dass dem Benutzer ein Bild des Lagerbereichs bereitgestellt wird, zum Beispiel durch Anzeige auf einem Display des Inkubators. Auf diese Weise kann der Benutzer sich unmittelbar ein "Bild" davon machen, ob die Belegung des Lagerbereichs das Einstellen weiterer Objekte erlaubt, und ob der entsprechende Lagerbereich ein von ihm eingestelltes und nun zu prüfendes oder zu entnehmendes Objekt -soweit für den Benutzer identifizierbar- enthält und wo dieses zu finden sein könnte. Ferner kann die Information auch weiter aufbereitet werden, indem mittels automatisierter Bildauswertung Daten über freien Lagerplatz in diesem Lagerbereich gewonnen und dem Benutzer mitgeteilt werden. Auch eine Erkennung von Objektklassen und Objektindividualmerkmalen wird durch das Verfügen über die Bilddaten ermöglicht.

Der Inkubator ist ein Laborgerät bzw. ein Laborinkubator. Ein Inkubator bezeichnet insbesondere ein Laborgerät mit einer Inkubatorkammer, deren Atmosphäre vom Inkubator auf eine vorgegebene Zieltemperatur regelbar ist bzw. geregelt wird. Insbesondere handelt es sich um ein Laborgerät, mit dem kontrollierte Klimabedingungen für verschiedene biologische Entwicklungs- und Wachstumsprozesse geschaffen und erhalten werden können. Der Inkubator kann ein Schüttelinkubator (Shaker), also ein Inkubator mit einer Bewegungseinrichtung zur Bewegung von in der Inkubatorkammer angeordneten Objekten, sein oder diesen beinhalten, und kann ein Microbial incubator (auch ohne CO₂) sein. Der Inkubator kann insbesondere als eine Zellkultivierungsvorrichtung ausgebildet sein. Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N₂-Gehalts des Gases und/oder eine Einstelleinrichtung zur Einstellung der Luftfeuchtigkeit in der Inkubatorkammer des Inkubators. Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden. Eine mit Wasser gefüllte Wanne in der Inkubatorkammer kann geheizt oder gekühlt werden, um über die Verdunstung die Luftfeuchtigkeit einzustellen. Alternativ und/oder zusätzlich kann ein Wasserverdampfer als Bestandteil des Inkubators vorgesehen sein, mittels dem die Luftfeuchtigkeit in der Atmosphäre der Inkubatorkammer eingestellt wird. CO₂-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen des mindestens einen Zellkulturbehälters aufweisen. Der erfindungsgemäße Inkubator ist insbesondere nicht ein Bioreaktor oder Fermentor.

Der Inkubator kann eine Sensoreinrichtung aufweisen. Eine Sensoreinrichtung weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Ein Inkubator weist vorzugsweise eine bzw. eine einzige Inkubatorkammer auf. Diese kann in Kompartimente unterteilt sein. Kompartimente können durch -insbesondere gelochte-Lagerplatten getrennt sein, wobei insbesondere ein Gasaustausch zwischen den Kompartimenten ermöglicht ist. Eine Lagerplatte, insbesondere deren untere Seite, kann zum Halten der Kameraeinrichtung eingerichtet sein und kann insbesondere eine Halterung für die Kameraeinrichtung aufweisen. Eine Lagerplatte, insbesondere deren untere Seite, kann zum Halten der Beleuchtungseinrichtung eingerichtet sein und kann insbesondere eine Halterung für die Beleuchtungseinrichtung aufweisen. Die Beleuchtungseinrichtung oder deren Halterung kann aber auch an anderer Stelle der Inkubatorkammer angeordnet bzw. montiert sein, z.B. an einer inneren Seitenwand der Inkubatorkammer, oder an Boden oder Deckenwand. Eine Halterung für die Beleuchtungseinrichtung kann ein Schienensystem, einen von der Steuereinrichtung gesteuerten Roboterarm, und/oder (einen) Magnet(en) aufweisen.

Die Inkubatorkammer weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Diese Kammeröffnung ist durch ein mit der Inkubatorkammer beweglich verbundenes Verschlusselement verschließbar, insbesondere eine mittels Scharnier an der Inkubatorkammer beweglich montierte Inkubatortüre, insbesondere eine oder mehrere Kammertüren. Ein Inkubator kann eine oder mehrere Innentüren aufweisen, die insbesondere transparent sein können, und kann eine -insbesondere nicht transparente- Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert. Vorzugsweise werden vom Bilderfassungssystem Bilder bei geschlossener Inkubatortüre bzw. Außentüre erfasst, damit ein Umgebungslicht keinen Einfluss auf die Beleuchtung des Lagerbereichs nimmt, die vorzugsweise ausschließlich durch die Beleuchtungseinrichtung erfolgt. Dies führt zu besonders gut reproduzierbaren, gut vergleichbaren und einfach durch Bildverarbeitungsalgorithmen auswertbaren Bildaufnahmen. Dennoch ist es auch möglich, dass die Bildaufnahmen bei geöffneter Inkubatortüre erstellt werden.

In der verschlossenen Position der Kammeröffnung ist das Innere der Inkubatorkammer gegen die Umgebung vorzugsweise in der Weise isoliert, so dass im Inneren eine gewünschte, vom Inkubator gesteuerte Temperatur bzw. Atmosphäre einstellbar, insbesondere regelbar ist. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, Kupfer, Messing, oder ein Kunststoff, insbesondere ein Verbundkunststoff. Dadurch wird die Reinigung/ Desinfektion des Kammerinneren erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Kabelverbindung aus dem Inneren der Inkubatorkammer an deren Außenseite oder in die Umgebung des Inkubators aufweisen.

Vorzugsweise sind die Oberflächen der Inkubatorinnenwände nicht-glänzend bzw. nicht-reflektierend ausgestaltet, insbesondere durch Verwendung einer matten Oberfläche. Die Oberfläche der Inkubatorinnenwand kann durch eine Oberflächenbehandlung mattiert sein. Die Oberflächenbehandlung kann insbesondere Schleifen mit einem Schleifmittel sein, das insbesondere eine bestimmte Körnung aufweisen kann. Die Oberflächenbehandlung kann insbesondere Bestrahlen mit einem Strahlmittel, insbesondere Sand oder Glasperlen insbesondere per Druckluft, sein, das insbesondere eine bestimmte Körnung bzw. einen charakteristischen Partikeldurchmesser aufweisen kann. Dadurch können störende Reflexionen in einer Bildaufnahme verhindert bzw. reduziert werden.

Eine typische Größe des Inneren einer Inkubatorkammer liegt zwischen 50 und 400 Litern.

Der Inkubator kann genau eine Inkubatorkammer aufweisen, kann aber auch mehrere Inkubatorkammern aufweisen, deren Atmosphäre (Temperatur, relative Gaskonzentration, Luftfeuchte) insbesondere individuell oder gesammelt einstellbar sein kann. Ein Inkubator kann mehrere Inkubatorkammern aufweisen, die jeweils eine eigene Kammeröffnung und eine eigene Kammertüre zum Verschließen der Kammeröffnung aufweisen können.

Der Inkubator kann ein Gehäuse aufweisen, dass die Inkubatorkammer teilweise oder vollständig umgibt. Das Gehäuse kann im Wesentlichen quaderförmig ausgebildet sein, und kann insbesondere derart ausgebildet sein, dass der Inkubator stapelbar ist.

Ein Lagerbereich des Inkubators ist insbesondere durch eine Lagerplatte, insbesondere einen Regalblecheinsatz und/oder eine bewegte Plattform realisiert, die/der insbesondere aus Edelstahl oder Kuper oder ähnlichem bestehen können oder dieses Material aufweisen. Eine Lagerplatte dient als Bodenplatte, insbesondere als Zwischenbodenplatte. Die Lagerplatte kann der Inkubatorkammer entnehmbar sein ("Lagerplatteneinsatz") oder kann fest eingesetzt mit dieser verbunden sein. Die Inkubatorkammer kann Halteabschnitte oder einen Haltrahmen zum Halten eines oder mehrerer Lagerplatteneinsätze oder einsetzbarer Instrumente aufweisen. Eine Lagerplatte kann an ihrer Unterseite zum Halten einer Kamera eingerichtet sein, insbesondere eine Halterung für diese Kamera aufweisen. Alternativ oder zusätzlich kann mindestens eine der Innenwände der Inkubatorkammer zum Halten eines oder mehrerer Lagerplatteneinsätze oder einsetzbarer Instrumente, insbesondere der mindestens einen Kamera, eingerichtet sein. Dazu kann eine in die Wand integrierte Haltestruktur vorgesehen sein, insbesondere ein oder mehrere Vorsprünge, Rinnen oder Stege. Eine Lagerplatte vergrößert die verfügbare Lagerfläche in der Inkubatorkammer.

Vorzugsweise sind im Wesentlichen alle Oberflächen oder mindestens eine Oberfläche der mindestens einen Lagerplatte nicht-glänzend bzw. nicht-reflektierend ausgestaltet, insbesondere durch Verwendung einer matten Oberfläche. Die Oberfläche der Inkubatorinnenwand kann durch eine Oberflächenbehandlung mattiert sein. Die Oberflächenbehandlung kann insbesondere Schleifen mit einem Schleifmittel sein, das insbesondere eine bestimmte Körnung aufweisen kann. Die Oberflächenbehandlung kann insbesondere Bestrahlen mit einem Strahlmittel, insbesondere Sand oder Glasperlen insbesondere per Druckluft, sein, das insbesondere eine bestimmte Körnung bzw. einen charakteristischen Partikeldurchmesser aufweisen kann. Dadurch können störende Reflexionen in einer Bildaufnahme verhindert bzw. reduziert werden.

Ein Halterahmen für die mindestens eine Lagerplatte ist ebenfalls vorzugsweise aus einem nicht-korrosiven Material gefertigt, vorzugsweise Edelstahl. Der Halterahmen ist vorzugsweise als stehendes Objekt ausgelegt, indem er mindestens einen Sockelabschnitt aufweist, der auf der Bodenwand der Inkubatorkammer ruht. Er kann sich aber auch an den Seitenwänden der Inkubatorkammer abstützen und/oder an der Deckenwand der Inkubatorkammer aufgehängt sein.

Eine Lagerplatte erstreckt sich vorzugsweise - und insbesondere im Wesentlichen vollständig- über einen horizontalen Querschnitt der Inkubatorkammer.

Vorzugsweise weist ein Inkubator mindestens zwei Lagerplatten auf, die übereinander angeordnet sind. Der Volumenbereich zwischen zwei Lagerplatten, bzw. zwischen einer Bodenwand der Inkubatorkammer und einer untersten Lagerplatte oder zwischen einer Deckenwand der Inkubatorkammer und einer obersten Lagerplatte kann als Lagerkompartiment bezeichnet werden. Ein Lagerkompartiment kann insgesamt als Lagerbereich aufgefasst werden. Die zum Lagern geeignete Oberfläche einer Lagerplatte kann als Lagerbereich aufgefasst werden. Die Höhe eines Lagerkompartiments ist vorzugsweise so bemessen, dass ein Objekt einer bestimmten Maximalhöhe (gemessen senkrecht zur planaren Oberfläche einer Lagerplatte) bzw. ein Objektstapel von Objekten einer bestimmten Maximalhöhe des Stapels auf der Lagerplatte abstellbar ist. Die Maximalhöhe kann insbesondere im Wesentlichen dem Abstand zweier Lagerplatten entsprechen.

Der Abstand zwischen zwei Lagerplatten bzw. die Maximalhöhe beträgt insbesondere zwischen 5 cm und 70 cm, vorzugsweise zwischen 5 cm und 65 cm, vorzugsweise zwischen 5 cm und 60 cm, vorzugsweise zwischen 5 cm und 50 cm, vorzugsweise zwischen 10 cm und 30 cm, vorzugsweise zwischen 10 cm und 20 cm, vorzugsweise zwischen 12 cm und 18 cm. Die Maximalhöhe kann insbesondere bis 150 cm betragen. Der Abstand zwischen zwei Lagerplatten ist mittels einer variablen Halteeinrichtung für Lagerplatten vorzugsweise vom Benutzer wählbar.

Ein in das Innere der Inkubatorkammer einsetzbares Instrument, insbesondere eine Kamera, kann als Modul ausgebildet sein und ermöglicht die Durchführung von automatisierten Beobachtungen im Inneren, vorzugsweise auch bei geschlossener Inkubatortüre.

Die Kameraeinrichtung, oder deren mindestens eine Kamera, ist vorzugsweise an einer Lagerplatte angeordnet, vorzugsweise unterhalb einer Lagerplatte angeordnet oder anordenbar und insbesondere dort an dieser befestigt oder befestigbar. Vorzugsweise ist mindestens eine Kamera an der Unterseite einer Lagerplatte montiert bzw. montierbar, insbesondere in einem geometrischen Zentrum der Unterseite, insbesondere im Kreuzungspunkt der Diagonalen einer rechteckförmigen Unterseite.

Vorzugsweise ist eine Kamera -oder sind mehrere Kameras- an der Unterseite eines Regalblecheinsatzes in der Inkubatorkammer montiert bzw. montierbar, oder an einer Unterseite der oberen Innenwand (Deckenwand) der Inkubatorkammer, vorzugsweise jeweils vertikal oberhalb des geometrischen Zentrums des Lagerbereichs, den die Kamera überwacht. Eine oder mehrere Kameras können aber auch an inneren Seitenwänden der Inkubatorkammer oder an einem Halterahmen angeordnet bzw. befestigt oder anordenbar/befestigbar sein.

Vorzugsweise ist die mindestens eine Kamera so eingerichtet und angeordnet, dass sie einen Bildwinkel aufweist, in der Regel gemessen in der Bilddiagonalen oder alternativ dazu auch in der Bildvertikalen oder Bildhorizontalen, der zwischen 90° und 210°, vorzugsweise zwischen 120° und 180°, und vorzugsweise zwischen 160° und 180° beträgt.

Eine Kamera des Bilderfassungssystems kann eine Weitwinkeloptik, insbesondere eine Fischaugenoptik aufweisen, deren Bildwinkel in der Bilddiagonalen zwischen 120° und 230° betragen kann.

Vorzugsweise ist genau eine Kamera an der Unterseite einer Lagerplatte vorgesehen, die insbesondere einen der oben genannten Bildwinkel aufweist.

Der "Sichtbereich" oder Sichtfeld (englisch field of view, FOV) einer Kamera kann insbesondere so definiert werden, dass er einen bestimmten Bildwinkel aufweist, z.B. einen Bildwinkel gemäß einem der oben definierten Bereiche, der zur Darstellung eines von diesem Sichtwinkel abhängigen Bildinhalts führt, oder kann durch einen in der Bildvertikalen gemessenen Bildwinkel und einen in der Bildhorizontalen gemessenen Bildwinkel definiert sein. Das Seitenverhältnis des Bildes kann insbesondere eines der folgenden Formate sein: 4:3, 3:2, 16:9, 1:1.

Vorzugsweise ist an der Unterseite einer Lagerplatte mindestens eine Kameravorzugsweise genau eine Kamera- angeordnet, deren Sichtbereich vorzugsweise mehr als X% der Ablagefläche einer im Sichtbereich der mindestens einen Kamera liegenden Lagerplatte erfasst. X ist, jeweils vorzugsweise 20, 30, 40, 50, 60, 70, 80, 90, 100. In anderen Worten: dass durch diese -genau eine oder mindestens eine-Kamera aufgenommene -genau eine oder mindestens eine- Bild zeigt vorzugsweise mehr als X% der Ablagefläche einer im Sichtbereich der mindestens einen Kamera liegenden Lagerplatte. Es können z.B. mehrere Kameras vorgesehen sein, die zusammen den gesamten Lagerbereich, also 100% der Ablagefläche, oder den Anteil X erfassen. Es ist vorzugsweise genau eine Kamera vorgesehen, die den gesamten Lagerbereich, oder den Anteil X erfasst. Je größer bzw. vollständiger der Sichtbereich ist, desto zuverlässiger bzw. effizienter kann der Lagerbereich abgebildet und das Bild ausgewertet werden.

Vorzugsweise sind an der Unterseite einer Lagerplatte mindestens eine Kamera - vorzugsweise genau eine Kamera- angeordnet, deren Sichtbereich in einem Kompartiment des Inkubators liegt und der, vorzugsweise zusätzlich zu der Ablagefläche (ein Teil oder die gesamte Ablagefläche) auf der Lagerplatte, vorzugsweise mehr als Y% der Wandfläche einer, das Kompartiment begrenzenden Kompartimentwand erfasst, die durch einen Innenwandabschnitt der Innenwand des Inkubators gebildet wird. Y ist, jeweils vorzugsweise 20, 30, 40, 50, 60, 70, 80, 90, 100. In anderen Worten: dass durch diese -genau eine oder mindestens eine- Kamera aufgenommene -genau eine oder mindestens eine- Bild zeigt vorzugsweise mehr als Y% der der Wandfläche einer (oder aller), das Kompartiment begrenzenden Kompartimentwand. Es können z.B. mehrere Kameras vorgesehen sein, die zusammen die gesamte Fläche aller Kompartimentwände, also 100% der Innenwandfläche eines Kompartiments, oder den Anteil Y erfassen. Es ist vorzugsweise genau eine Kamera vorgesehen, die die gesamte Fläche aller Kompartimentwände, oder den Anteil Y, erfasst. Durch den entsprechend großen Sichtbereich können insbesondere auch Objekte oder Objektstapel erfasst werden, die an einem Rand des planaren Lagerbereichs der Lagerplatte positioniert sind.

Die Datenverarbeitungseinrichtung ist vorzugsweise dazu programmiert, dass mittels der Kamera aufgenommene Bild automatisch zu beschneiden, so dass sich ein effektiver Bildwinkel ergibt, der kleiner ist als der die Kamera spezifizierende Bildwinkel bzw., dass sich so ein effektiver Sichtbereich ergibt, der kleiner ist als der die Kamera spezifizierende Sichtbereich.

Die Kamera des Inkubators ist insbesondere geeignet, um in der jeweiligen Inkubatoratmosphäre über einen Zeitraum von mehreren Monaten oder Jahren zuverlässig zu arbeiten, bzw. um während der unter Standardbedingungen (Raumtemperatur) gemessenen Lebensdauer zuverlässig zu arbeiten. Nicht jede Kamera ist geeignet, um in einer Inkubatoratmosphäre zu funktionieren. Eine mögliche, kommerziell erhältliche Kamera ist die 5MP Weitwinkel Camera für Raspberry Pi, www.joy-it.net, erhältlich bei Conrad Electronic SE, Deutschland, und/oder eine andere Kamera in Kombination mit einer Weitwinkellinse, z.B. kommerziell erhältlich die "Industrial lens HAL 250 2.3", Entaniya Co.,Ltd., Japan. Alternativ kann eine Hülleinrichtung für die mindestens eine Kamera vorgesehen sein, um diese gegenüber der Inkubatoratmosphäre abzuschirmen bzw. zu isolieren, wobei diese Hülleinrichtung insbesondere transparente Bereiche oder ein transparentes Fenster aufweist bzw. transparent ist, um die Bildaufnahmen durch die transparenten Bereich zu ermöglichen.

Vorzugsweise weist die Kameraeinrichtung mindestens einen optischen Filter auf, mit dem das auf die Kamera einfallende Licht gefiltert wird. Dadurch kann die Qualität der Bildaufnahme optimiert werden, insbesondere hinsichtlich einer nachgeordneten digitalen Bildverarbeitung und -auswertung. Vorzugsweise weist die Kameraeinrichtung mindestens einen Polfilter auf, mit dem das auf die Kamera einfallende Licht gefiltert wird. Dadurch können Reflektionen in der Bildaufnahme reduziert werden, die aus Reflektionen des Lichts der Beleuchtungseinrichtung an Objekten im Lagerbereich, Elementen des Lagerbereichs oder der Inkubatorkammer und/oder Innenwänden der Inkubatorkammer entstehen können, insbesondere hinsichtlich einer nachgeordneten digitalen Bildverarbeitung und -auswertung. Der Polfilter ist vorzugsweise ein zirkularer Polfilter, kann aber auch linear sein. Unerwünschte Reflexionen von glatten, nichtmetallischen Oberflächen (z. B. der Kunststoffoberfläche von Zellkulturbehältern) lassen sich mittels Polfilter unterdrücken. An nichtmetallischen Oberflächen wird Licht mit senkrechter Polarisation merklich stärker reflektiert, insbesondere wenn der Austrittswinkel zur Oberfläche etwa 30° bis 40° beträgt, also nahe dem Brewster-Winkel liegt. Wenn der Polarisationsfilter geeignet ausgerichtet ist, werden die reflektierten Lichtwellen unterdrückt, so dass der unpolarisierte Hintergrund nicht von den Reflexionen überstrahlt wird. Vorzugsweise werden bei Verwendung von Polarisation die aufzunehmenden Objekte, insbesondere Zellkulturbehälter, -insbesondere in direkter Linie- zwischen der Beleuchtungseinrichtung und der mindestens einen Kamera angeordnet.

Vorzugsweise weist die Kameraeinrichtung einen ersten Polfilter auf und die Beleuchtungseinrichtung einen zweiten Polfilter auf, wobei insbesondere der erste und zweite Polfilter gedreht zueinander verwendet werden. Auf diese Weise wird zunächst ein Teil des Lichtes der Beleuchtungseinrichtung durch den Polfilter davor ausgesperrt. Der Polfilter vor der Kamera wird dabei bezüglich des Polfilters der Beleuchtungseinrichtung so eingestellt bzw. gedreht zum einfallenden Licht angeordnet, dass er nun auch den anderen Teil des von der Beleuchtungseinrichtung abgegebenen Lichtes aussperrt. Übrig bleibt idealerweise nur noch diffuses Licht. Infolge dessen werden Reflexionen, auch von metallischen Oberflächen, abgemildert bzw. ganz ausgelöscht. Vorteilhaft ist dies insbesondere hinsichtlich einer nachgeordneten digitalen Bildverarbeitung und -auswertung, insbesondere hinsichtlich einer Umrisserkennung von Objekten mittels Bildverarbeitung.

Die Beleuchtungseinrichtung weist vorzugsweise mindestens eine Lichtquelle, insbesondere LED, auf. Vorzugsweise weist die Beleuchtungseinrichtung mindestens zwei Lichtquellen oder mehr mit jeweils unterschiedlichem Emissionsspektrum auf, also unterschiedlichen Farben, z.B. rot, grün, blau. Auf diese Weise kann die Bildqualität optimiert werden, insbesondere hinsichtlich einer nachgeordneten digitalen Bildverarbeitung und -auswertung, insbesondere hinsichtlich einer Umrisserkennung von Objekten mittels Bildverarbeitung.

Die Beleuchtungseinrichtung kann mindestens eine Lichtquelle aufweisen, deren emittiertes Licht Wellenlängen größer als das von sichtbarem Licht aufweist bzw. daraus besteht, insbesondere deren Emissionsspektrum im Infrarotbereich mit insbesondere einer Wellenlänge zwischen 780 nm und 1 mm, insbesondere im Nahinfrarot (780 nm bis 3000 nm) oder mittlerem Infrarot (3000 nm bis 50000 nm) liegt oder einen solchen Infrarotbereich enthält. Die Kameraeinrichtung weist in diesem Fall mindestens eine Kamera bzw. einen Kamerasensor auf, der zur Erfassung von entsprechendem Licht, insbesondere Infrarotlicht, geeignet ist. Die Beleuchtungseinrichtung kann mindestens eine Lichtquelle aufweisen, deren emittiertes Licht Wellenlängen kleiner als das von sichtbarem Licht aufweist bzw. daraus besteht. Die Kameraeinrichtung weist in diesem Fall mindestens eine Kamera bzw. einen Kamerasensor auf, der zur Erfassung von entsprechendem Licht geeignet ist.

Vorzugsweise sind mindestens zwei Lichtquellen in einem Abstand zueinander angeordnet. Dadurch kann das Sichtfeld der Kamera(s) homogener ausgeleuchtet werden und die Intensität einzelner, lichtrichtungsabhängiger Reflexionsbereiche kann reduziert werden. Dies ist vorteilhaft insbesondere hinsichtlich einer nachgeordneten digitalen Bildverarbeitung und -auswertung, insbesondere hinsichtlich einer Umrisserkennung von Objekten mittels Bildverarbeitung.

Vorzugsweise weist die Beleuchtungseinrichtung mindestens eine Lichtquelle auf, die an einer Unterseite einer Lagerplatte angeordnet bzw. befestigt oder anordenbar/befestigbar ist. Vorzugsweise weist die Beleuchtungseinrichtung mindestens zwei oder mehr Lichtquellen auf, die an unterschiedlichen Positionen entlang einer Unterseite einer Lagerplatte angeordnet bzw. befestigt oder anordenbar/befestigbar ist.

Vorzugsweise weist die Beleuchtungseinrichtung mindestens einen optischen Filter auf, durch den das von der Beleuchtungseinrichtung emittierte Licht teilweise oder vollständig gefiltert wird. Der optische Filter kann ein Polfilter sein, der insbesondere auf einen Polfilter der Kameraeinrichtung abgestimmt sein, um insbesondere die optimale gewünschte Filterwirkung zu entfalten.

Vorzugsweise weist die Beleuchtungseinrichtung mindestens einen Lichtdiffusor auf, wodurch die Beleuchtungseinrichtung ein diffuses Licht emittiert. Ein Lichtdiffusor kann z.B. eine milchige Plexiglasplatte sein oder diese aufweisen. Durch den Lichtdiffusor können insbesondere harte Schatten und Reflexionen reduziert bzw. verhindert werden, was insbesondere hinsichtlich einer nachgeordneten digitalen Bildverarbeitung und -auswertung vorteilhaft ist.

Vorzugsweise weist die Bilderfassungseinrichtung, insbesondere die Kameraeinrichtung und/oder die Beleuchtungseinrichtung mindestens eine Blende auf, vorzugsweise eine Aperturblende, die insbesondere variablen Durchmesser hat, z.B. eine Irisblende, um den Lichtfluss der Beleuchtung oder den in die Kamera eintretenden Lichtfluss zu kontrollieren.

Vorzugsweise weist die Bilderfassungseinrichtung, insbesondere die Kameraeinrichtung und/oder die Beleuchtungseinrichtung mindestens eine oder mehrere optische Linsen auf.

Vorzugsweise weist die Bilderfassungseinrichtung, insbesondere die Kameraeinrichtung und/oder die Beleuchtungseinrichtung mindestens ein Objektiv auf, vorzugsweise ein Weitwinkelobjektiv, vorzugsweise ein weitwinkliges Fischaugenobjektiv.

Vorzugsweise weist die Bilderfassungseinrichtung einen Zeitgeber auf. Vorzugsweise ist die Datenverarbeitungseinrichtung dazu programmiert mindestens eine oder mehrere Lichtquellen der Beleuchtungseinrichtung in einer vorbestimmten zeitlichen Sequenz zu aktivieren und insbesondere nach jeweils vorbestimmter Aktivitätszeit wieder zu deaktivieren und/oder alle oder mehrere Lichtquellen gleichzeitig zu aktivieren, und vorzugsweise dazu programmiert, dass die Kameraeinrichtung mehrere Bilder des Lagerbereichs erfasst, die jeweils nacheinander und insbesondere synchron zu den Aktivitätszeiten der Beleuchtung erfasst wurden.

Vorzugsweise ist das Bilderfassungssystem dazu eingerichtet, den Zeitpunkt des Eintritts eines Objektes in die Inkubatorkammer und/oder den Zeitpunkt der Entnahme eines Objektes aus der Inkubatorkammer zu erfassen und zu speichern.

Vorzugsweise ist das Bilderfassungssystem dazu eingerichtet,
- mittels der Beleuchtungseinrichtung mindestens ein oder zwei auf diesem Lagerbereich angeordnete Objekte zu beleuchten,
- mittels der Kameraeinrichtung ein Bild der mindestens ein oder zwei Objekte auf diesem Lagerbereich zu erfassen, und
- das Bild der mindestens ein oder zwei Objekte mittels der Datenverarbeitungseinrichtung in Form von Bilddaten in der Datenspeichereinrichtung zu speichern. Auf diese Weise werden verschiedene Einsatzmöglichkeiten der Bilddaten ermöglicht, insbesondere: die Unterscheidung von Objekten im Lagerbereich, insbesondere: dem ersten und zweiten Objekt unterschiedliche Identifikationsdaten zuzuordnen; die Objekte zu zählen; die Objektklasse zu erkennen; Individualmerkmale zu analysieren, zu speichern, zu erkennen; Objekte bei Bewegung zu verfolgen; Zeitpunkt des Eintritts oder der Entnahme des Objektes erkennen und speichern.

Vorzugsweise ist die Datenverarbeitungseinrichtung dazu programmiert,
- mittels Auswertung der Bilddaten das in dem Bild dargestellte erste Objekt und zweite Objekt zu unterscheiden, insbesondere: dem ersten und zweiten Objekt unterschiedliche Identifikationsdaten zuzuordnen; die Objekte zu zählen; die Objektklasse zu erkennen; Individualmerkmale zu analysieren, speichern, erkennen; Objekte bei Bewegung zu verfolgen, insbesondere mittels Bildverarbeitungsalgorithmen die Umrisse des ersten und zweiten Objektes in dem Bild zu erfassen, und
- insbesondere Informationen über das erste und zweite Objekt, insbesondere die Bounding Boxen und/oder Umrisse des ersten und zweiten Objektes, in Form von Objektdaten in der Datenspeichereinrichtung zu speichern.

Vorzugsweise ist in einer bevorzugten Ausführungsform die Beleuchtungseinrichtung dazu eingerichtet, und insbesondere ist die Datenverarbeitungseinrichtung dazu programmiert, dass die Beleuchtungseinrichtung in mindestens zwei verschiedenen Beleuchtungsmodi betrieben wird, und das Bilderfassungssystem ist vorzugsweise dazu eingerichtet, und insbesondere ist die Datenverarbeitungseinrichtung dazu programmiert,
- den Lagerbereich der Inkubatorkammer mittels der Beleuchtungseinrichtung
   i) zunächst in einem ersten Beleuchtungsmodus und
   ii) danach in einem davon verschiedenen zweiten Beleuchtungsmodus zu beleuchten,
- mindestens ein Bild des Lagerbereichs während der Beleuchtung sowohl mittels des ersten als auch des zweiten Beleuchtungsmodus mittels der Kameraeinrichtung zu erfassen, und
- das mindestens eine Bild in Form von Bilddaten bereitzustellen, die sowohl während des ersten als auch des zweiten Beleuchtungsmodus erfasste kombinierte Bildinformationen enthalten,
wobei die Datenverarbeitungseinrichtung dazu programmiert ist, ein Bildauswertungsprogramm auszuführen, das die kombinierten Bildinformationen aus den Bilddaten gewinnt.

Durch diese Ausführungsform kann insbesondere die Qualität der Bilderfassung des Lagerbereichs verbessert bzw. optimiert werden, was insbesondere hinsichtlich einer nachgeordneten Bildverarbeitung, insbesondere -auswertung vorteilhaft ist, insbesondere zur Erfassung der Bounding Box(en) und/oder des Umrisses/der Umrisse von einem oder mehreren Objekten bzw. Zellkulturbehältern.

Ein erster Beleuchtungsmodus und ein zweiter Beleuchtungsmodus können sich insbesondere dadurch unterscheiden, dass verschiedene Lichtquellen verwendet werden, und/oder an unterschiedlichen Positionen angeordnete Lichtquellen, und/oder verschiedene Belichtungszeiten der Lichtquellen, und/oder verschiedene Emissionsspektren bzw. Lichtfarben, und/oder verschiedene Lichtintensitäten. Durch die unterschiedlichen Beleuchtungsmodi kann insbesondere die Qualität der Bilderfassung des Lagerbereichs verbessert bzw. optimiert werden, was insbesondere hinsichtlich einer nachgeordneten Bildverarbeitung, insbesondere -auswertung vorteilhaft ist, insbesondere zur Erfassung des Umrisses/der Umrisse von einem oder mehreren Objekten bzw. Zellkulturbehältern.

Vorzugsweise enthält das mindestens eine Bild des Lagerbereichs mindestens ein erstes Bild des Lagerbereichs und ein davon verschiedenes zweites Bild des Lagerbereichs, wobei das erste Bild im ersten Beleuchtungsmodus erfasst wird und das zweite Bild im zweiten Beleuchtungsmodus erfasst wird, und das erste Bild in Form von ersten Bilddaten und das zweite Bild in Form von zweiten Bilddaten bereitgestellt wird,
wobei insbesondere die Datenverarbeitungseinrichtung und/oder das Bildauswertungsprogramm so programmiert sind, dass
- die ersten Bilddaten und die zweiten Bilddaten kombiniert werden, um kombinierte Bilddaten zu erhalten, die insbesondere durch eine Addition und/oder Mittelung von ersten und zweiten Bilddaten entsteht, und
- die kombinierten Informationen aus den kombinierten Bilddaten gewonnen werden.

Durch diese Ausführungsform kann insbesondere die Qualität der Bilderfassung des Lagerbereichs verbessert bzw. optimiert werden, was insbesondere hinsichtlich einer nachgeordneten Bildverarbeitung, insbesondere -auswertung vorteilhaft ist, insbesondere zur Erfassung der Positionen von einem oder mehreren Objekten bzw. Zellkulturbehältern im Bild des Lagerbereichs mittels Bounding-Box-Algorithmen.

Eine typischer Programmcode zur bildverarbeitenden Objektverfolgung, der vorzugsweise zum Einsatz kommt, basiert auf der Auswertung der zeitlichen Abfolge von Bildern. Ein typischer Programmcode zur Objektverfolgung verwendet eine "Bounding Box" als Ausgabeformat, um ein Objekt in einem Bild zu identifizieren, dessen Kollisionsgrenzen festzulegen und insbesondere zu lokalisieren. In der digitalen Bildverarbeitung bezeichnet die "Bounding Box" die Koordinaten des rechteckigen Rahmens, der ein im digitalen Bild gezeigtes Objekt größtenteils oder vollständig umschließt. Durch die Verwendung von Bounding Boxes bei der Objektverfolgung wird diese effizienter, da die Bildauswertung mittels eines solchen numerischen Hilfsmittels, insbesondere im Vergleich mit Algorithmen der Umrisserkennung von Objekten weniger Rechenschritte und damit eine geringere Rechenleistung erfordert. Zudem lassen sich die entsprechenden Algorithmen mithilfe von spezialisierten Grafikprozessoren (GPUs) effizient und kostengünstig ausführen. Geeignete Programmierschnittstellen (APIs) zur Objektverfolgung mittels Bounding Boxes stehen in der Programmbibliothek OpenCV unter den Bezeichnungen BOOSTING, CSRT, GOTURN, KCF, MEDIANFLOW, MOSSE, MIL, TLD zur Verfügung. Entsprechend stehen in OpenCV Programmbibliotheken ("MultiTracker") für das zeitgleiche Verfolgen von mehreren Objekten ("multiple object tracking") zur Verfügung. Als Alternative dazu sind Deep Learning Algorithmen zum Multi-Object Tracking (MOT) nach dem "tracking-by-detection" Prinzip bekannt.

Es ist aber auch möglich und bevorzugt, dass zur Objektverfolgung eine Ermittlung der Kontur des zu verfolgenden Objektes im Bild durchgeführt wird, und insbesondere der Trennung von Objekt (Vordergrund) und Hintergrund durch Hintergrundsubtraktion.

Vorzugsweise werden eine Vielzahl (N>=10) von Beleuchtungsmodi verwendet, um entweder ein Bild zu erfassen oder mehrere Bilder zu erfassen, die dann in Form von kombinierten Bilddaten ein kombiniertes Bild mit kombinierter Bildinformation liefern. Vorzugsweise ist 2<= N <=300, vorzugsweise 10 <= N <= 300, vorzugsweise 100 <= N <= 300. Dabei kann vorzugsweise sein N<= 500 oder N<=1000.

Vorzugsweise enthält das mindestens eine Bild des Lagerbereichs ein mehrfach belichtetes Bild des Lagerbereichs, wobei das Bilderfassungssystem insbesondere dazu eingerichtet ist,
- das Bild des Lagerbereichs während der Beleuchtung sowohl mittels des ersten als auch des zweiten Beleuchtungsmodus mittels der Kameraeinrichtung zu belichten und zu erfassen, und
- das mehrfach belichtete Bild in Form der Bilddaten bereitzustellen.

Vorzugsweise enthält das mindestens eine Bild Informationen über im Lagerbereich angeordneten Objekte, insbesondere Informationen, wahlweise,
* über die Positionen der Objekte im Lagerbereich
* über die Außenkonturen der Objekte,
* über die Fläche der Objekte, gemessen in einer Ebene parallel zu einer planaren Fläche des Lagerbereichs,
* die von den Objekten nicht belegte Fläche des Lagerbereichs, gemessen in einer Ebene parallel zu einer planaren Fläche des Lagerbereichs.

Vorzugsweise weist die Beleuchtungseinrichtung mindestens eine erste und eine zweite Lichtquelle auf, die im ersten und zweiten Beleuchtungsmodus unterschiedlich betrieben werden, wobei insbesondere die erste und eine zweite Lichtquelle in einem Abstand oberhalb einer Lagerfläche des Lagerbereichs angeordnet sind, wobei insbesondere die erste und zweite Lichtquelle in einer Ebene, die parallel zu einer planaren Lagerfläche des Lagerbereichs liegt, versetzt angeordnet sind, wobei insbesondere der Lagerbereich eine planare Lagerfläche aufweist, wobei die erste Lichtquelle senkrecht oberhalb einer ersten Hälfte der Lagerfläche angeordnet ist und die zweite Lichtquelle senkrecht oberhalb einer zweiten Hälfte der Lagerfläche angeordnet ist, wobei insbesondere die Beleuchtungseinrichtung ein LED-Leuchtband mit mehreren LED-Lichtquellen aufweist, das in einer Ebene, die parallel zu einer planaren Lagerfläche des Lagerbereichs liegt, angeordnet ist, insbesondere in einem mäandernden Verlauf, einem spiralartigen Verlauf, insbesondere in einem zumindest abschnittsweise linearen Verlauf angeordnet ist, wobei insbesondere das Bilderfassungssystem eine, insbesondere programmierbare, elektronische Steuereinrichtung aufweist, die so eingerichtet bzw. programmiert ist, dass
∘ während einer Beleuchtungsphase des ersten Beleuchtungsmodus die erste Lichtquelle anders betrieben wird als während einer Beleuchtungsphase des zweiten Beleuchtungsmodus, und/oder
∘ während einer Beleuchtungsphase des ersten Beleuchtungsmodus die zweite Lichtquelle anders betrieben wird als während einer Beleuchtungsphase des zweiten Beleuchtungsmodus,
   insbesondere dass
∘ während einer Beleuchtungsphase des ersten Beleuchtungsmodus die erste Lichtquelle aktiv ist und während einer Beleuchtungsphase des zweiten Beleuchtungsmodus weniger aktiv (also mit geringerer Intensität abstrahlt) oder inaktiv ist, und/oder
∘ während einer Beleuchtungsphase des ersten Beleuchtungsmodus die zweite Lichtquelle weniger aktiv oder inaktiv ist und während einer Beleuchtungsphase des zweiten Beleuchtungsmodus aktiv ist,
   insbesondere dass
∘ während einer Beleuchtungsphase des ersten Beleuchtungsmodus die erste Lichtquelle mit einem anderen Emissionsspektrum betrieben wird als während einer Beleuchtungsphase des zweiten Beleuchtungsmodus, und/oder
∘ während einer Beleuchtungsphase des ersten Beleuchtungsmodus die zweite Lichtquelle mit einem anderen Emissionsspektrum betrieben wird als während einer Beleuchtungsphase des zweiten Beleuchtungsmodus.

Vorzugsweise ist insbesondere die mindestens eine Kamera in einem Abstand senkrecht oberhalb zu einer Lagerfläche des Lagerbereichs angeordnet, wobei vorzugsweise die mindestens eine Kamera eine Weitwinkeloptik aufweist, insbesondere eine Weitwinkel- bzw. Fischaugenobjektiv aufweist, wobei vorzugsweise genau eine Kamera vorgesehen ist, die in einem Abstand senkrecht oberhalb zu einem Zentrum der Lagerfläche des Lagerbereichs angeordnet ist.

Vorzugsweise ist das Bilderfassungssystem ein modularer, nämlich vom Benutzer wahlweise einsetzbarer Bestandteil des Inkubators, wobei insbesondere der Inkubator eine Steuereinrichtung und eine Temperiereinrichtung zur Regelung der Temperatur im Innenraum der Inkubatorkammer aufweist, wobei das Bilderfassungssystem eine andere Steuereinrichtung aufweist, die dazu eingerichtet ist, das Bilderfassungssystem zu steuern, insbesondere indem diese andere Steuereinrichtung die Datenverarbeitungseinrichtung des Bilderfassungssystems beinhaltet. Eine solche modulare Ausführungsform von Inkubator mit Bilderfassungssystem weist vorzugsweise zudem ein Dateninterface zum Inkubator auf, damit z.B. Bilddaten am Inkubatordisplay angezeigt werden können.

Vorzugsweise weist insbesondere der Inkubator eine Steuereinrichtung und eine Temperiereinrichtung zur Regelung der Temperatur im Innenraum der Inkubatorkammer auf, wobei insbesondere diese Steuereinrichtung dazu eingerichtet ist, das Bilderfassungssystem zu steuern, insbesondere indem diese Steuereinrichtung die Datenverarbeitungseinrichtung des Bilderfassungssystems beinhaltet. Es handelt sich um die integrale Ausführungsform von Inkubator mit Bilderfassungssystem.

Vorzugsweise weist der Inkubator ein Display auf und ist vorzugsweise dazu eingerichtet bzw. programmiert, auf dem Display
vorzugsweise das Bild, und/oder
vorzugsweise aus dem mindestens einem Bild entnommene Bildinformationen, und/oder
vorzugsweise ein die kombinierten Bildinformationen enthaltendes Bild des Lagerbereichs anzuzeigen.

Vorzugsweise ist das Bilderfassungssystem:
ein Objekterfassungssystem, in dem die Datenverarbeitungseinrichtung dazu programmiert ist, dass während der Bilderfassung des mindestens einen Bildes im Lagerbereich angeordnete mindestens eine Objekt mittels des Bildauswertungsprogramms zu erfassen,
insbesondere ein Objekterkennungssystem zum Erkennen des Objektes anhand individueller Eigenschaften und/oder zum Erkennen einer Objektklasse anhand objektspezifischer Klasseneigenschaften,
insbesondere ein Objektverfolgungssystem zur Verfolgung von Positionsänderungen des mindestens einen Objektes im Lagerbereich ausgehend von einer Startposition zur Erfassung von dessen Endposition.

"Nach unten" bezeichnet die Richtung der Gravitation, "nach oben" die entgegengesetzte Richtung. "Vertikal" bedeutet "entlang des Vektors der Gravitation", "horizontal" bedeutet senkrecht zur Vertikalen bzw. in einer planaren Ebene senkrecht zur Vertikalen. Im bestimmungsgemäßen Gebrauch sind Inkubatoren so angeordnet, dass die Oberseiten der planaren Lagerplatten horizontal liegen.

Vorzugsweise weist der Inkubator eine Behandlungseinrichtung zur Behandlung des mindestens einen Objekts, insbesondere Zellkulturbehälters auf. Der Begriff "Behandlung" bedeutet insbesondere, dass ein Objekt, insbesondere eine Zellkultur oder ein Zellkulturbehältnis bewegt, und/oder transportiert und/oder untersucht und/oder verändert wird, insbesondere physikalisch, chemisch, biochemisch oder auf andere Weise verändert wird. Eine Behandlungseinrichtung kann eine Bewegungseinrichtung sein, mittels der das Zellmedium in mindestens einem Zellkulturbehälter in Bewegung gehalten wird, vorzugsweise über ein Bewegungsprogramm, das vom Steuerprogramm gesteuert wird. Eine Bewegungseinrichtung kann eine Schüttel- oder Schwenkeinrichtung sein. Eine Bewegungseinrichtung weist vorzugsweise eine Trägereinrichtung auf, insbesondere eine Platte, auf der ein oder mehrere Zellkulturbehälter abgestellt und/oder fixiert werden. Eine Bewegungseinrichtung weist vorzugsweise eine Antriebseinrichtung auf, insbesondere im Fall einer Schütteleinrichtung beispielsweise einen Oszillatorantrieb, insbesondere in Kombination mit einem Exzenter, mittels dem das gewünschte Bewegungsprogramm umgesetzt wird. Eine Behandlungseinrichtung kann eine Schwenkeinrichtung sein, mittels der mindestens ein Zellkulturbehälter geschwenkt wird. Die Bestandteile der Schwenkeinrichtung können denen der Schütteleinrichtung entsprechen, sind aber für eine Schwenkbewegung eingerichtet.

Eine Behandlungseinrichtung kann auch eine Transporteinrichtung sein, mittels der mindestens ein Zellkulturbehälter in der Inkubatorkammer transportierbar ist.

Die Transporteinrichtung kann eine Lifteinrichtung sein, aufweisend eine Trägereinrichtung, auf der mindestens ein Objekt, insbesondere Zellkulturbehälter, Kamera, oder Lichtquelle, platzierbar ist. Die Transporteinrichtung bzw. Lifteinrichtung weist vorzugsweise einen Bewegungsmechanismus und/oder einen elektrisch ansteuerbaren Antriebsmechanismus zum Antreiben des Bewegungsmechanismus auf. Die Transporteinrichtung kann ferner einen beweglichen und elektrisch ansteuerbaren Greifarm zum Greifen und Halten mindestens eines Zellkulturbehälters sein. Die Transporteinrichtung kann ein Förderband oder ein Schienensystem zum Bewegen des mindestens einen darauf/daran platzierten Objektes aufweisen. Durch den Transport kann das mindestens eine Objekt in der Inkubatorkammer bewegt werden, insbesondere zu einer Bearbeitungsposition oder Aufnahmeposition, z.B. in einer Bearbeitungsstation, in der Inkubatorkammer, und von dieser Bearbeitungsposition oder Aufnahmeposition weg. Die Steuereinrichtung kann dazu eingerichtet sein, die Transporteinrichtung in Abhängigkeit von Informationen aus zuvor erfassten Bilddaten zu steuern.

Eine Behandlungseinrichtung kann auch eine Transporteinrichtung sein, mittels der mindestens eine Kamera der Kameraeinrichtung und/oder mindestens eine Lichtquelle in der Inkubatorkammer transportierbar ist. Die Transporteinrichtung kann insbesondere unterhalb bzw. unmittelbar unterhalb einer Lagerplatte angeordnet sein, und/oder unterhalb bzw. unmittelbar unterhalb einer Deckenwand der Inkubatorkammer. Durch eine bewegte bzw. bewegbare Lichtquelle können unterschiedliche Beleuchtungsmodi realisiert werden. Insbesondere kann der Beleuchtungsmodus einem Belegungszustand angepasst werden, z.B. um bei sehr dichter Belegung eines Lagerbereichs mit Objekten variabel eine geeignete Beleuchtungsrichtung zu realisieren. Durch mehrere Kameras, ebenso wie durch eine bewegte bzw. bewegbare Kamera können verschiedene Bilder bzw. Bildausschnitte des Lagerbereichs erstellt werden, die insbesondere durch digitale Bildverarbeitung zu einem Gesamtbild des Lagerbereichs kombiniert werden können. Im Falle einer bewegbaren Kamera besteht zudem die Möglichkeit, die Kameraposition einem Belegungszustand anzupassen, z.B. um bei sehr dichter Belegung eines Lagerbereichs mit Objekten variabel eine geeignete Beleuchtungsrichtung zu realisieren.

Insbesondere kann die Datenverarbeitungsrichtung dazu programmiert sein, die mindestens eine Kamera der Kameraeinrichtung und/oder mindestens eine Lichtquelle in der Inkubatorkammer mittels der Transporteinrichtung in vorbestimmter oder dynamisch adaptierter Weise zu transportieren. Beispielsweise kann die Datenverarbeitungsrichtung dazu programmiert sein, die mindestens eine Kamera der Kameraeinrichtung und/oder mindestens eine Lichtquelle in der Inkubatorkammer mittels der Transporteinrichtung zu unterschiedlichen Aufnahmepositionen zu transportieren, insbesondere das dort erstellte Bild jeweils mittels Bildverarbeitungsalgorithmus auszuwerten und insbesondere zu überprüfen, ob eine gewünschte Bildinformation, z.B. ein individuelles Merkmal eines Zellkulturbehälters, insbesondere ein Barcode, in ausreichender Qualität erfasst wurde, z.B. um den Barcode eindeutig zu lesen. Die Datenverarbeitungsrichtung kann dazu programmiert sein, die Kamera und/oder die Lichtquelle zu anderen Aufnahmepositionen zu bewegen, bis eine gewünschte Bildinformation erfasst wurde.

Die Kamera- und/oder Beleuchtungseinrichtung kann zudem an einer Transporteinrichtung befestigbar sein. Die Kamera- und/oder Beleuchtungseinrichtung kann an einem Positioniermechanismus befestigbar sein oder befestigt sein, mittels dem die Kamera- und/oder Beleuchtungseinrichtung in der Inkubatorkammer bewegbar und positionierbar ist. Der Positioniermechanismus kann einen beweglichen Roboterarm beinhalten und ist vorzugsweise elektrisch steuerbar, insbesondere durch ein Steuerprogramm der Steuereinrichtung. Auf diese Weise können unterschiedliche Aufnahmesituationen mit einer oder mit wenigen Kamera- und/oder Beleuchtungseinrichtungen nacheinander erfasst werden. Der Positioniermechanismus kann als in die Inkubatorkammer einsetzbares Bauteil ausgebildet sein. Die Energiezufuhr dieses Bauteils kann über eine Kabelverbindung mit dem Inkubator erfolgen, vorzugsweise über eine durch eine Wandöffnung, z.B. einen Port, oder über eine solche Kabelverbindung zu einer externen Spannungsquelle. Die Steuereinrichtung kann dazu eingerichtet sein, den Positioniermechanismus in Abhängigkeit von Zellüberwachungsdaten zu steuern.

Als Behandlungseinrichtung lässt sich auch die Temperiereinrichtung der Inkubatorkammer verstehen, mit der die Atmosphäre im Inneren der Inkubatorkammer auf den gewünschten Wert, insbesondere 37°C geregelt wird. Der Begriff Temperieren bezieht sich auf das Erhöhen und Senken der Atmosphärentemperatur durch Heizen und Kühlen. Vorzugsweise wird die Temperatur im Inneren über eine Temperaturänderung der Wände des Inkubators eingestellt. Temperatursensoren der entsprechenden Temperaturregeleinrichtung sind an wenigstens einer Position im Inneren und / oder außerhalb der Inkubatorkammer, insbesondere an einer Wand der Inkubatorkammer verteilt.

Der Inkubator weist vorzugsweise eine Benutzerschnittstelleneinrichtung auf, über die der Benutzer Daten an die Datenverarbeitungseinrichtung bzw. die Steuereinrichtung eingeben kann, und/oder über die Informationen an den Benutzer ausgegeben werden können. Vorzugsweise ist der Inkubator bzw. diese Benutzerschnittstelleneinrichtung dazu eingerichtet, dass der Benutzer mindestens einen Betriebsparameter zum Betreiben des Inkubators oder des Bilderfassungssystems an dieser Benutzerschnittstelleneinrichtung eingeben kann bzw. Informationen von dieser erhalten kann. Auf diese Weise kann eine einzige Benutzerschnittstelleneinrichtung vom Benutzer dazu verwendet werden, um den Inkubator und auch das mindestens eine Bilderfassungssystem zu beeinflussen, oder zu steuern, oder von diesen Informationen zu erhalten. Insbesondere kann das Bilderfassungssystem dazu eingerichtet sein, dem Benutzer auf eine mittels der Benutzerschnittstelleneinrichtung des Inkubators erfolgte Abfrage des Benutzers Positionsdaten oder freien Lagerplatz anzuzeigen, oder eine aus Positionsdaten abgeleitete Informationen (z.B. Identität des Benutzers, der die Positionsänderung herbeigeführt hat), insbesondere auch statistische Informationen, wie beispielsweise Häufigkeit und Zeitpunkt der Positionsveränderung eines Objektes (einer Probe) und / oder -insbesondere prozentual- verfügbarer freier Lagerplatz, und/oder mindestens ein optisches Abbild des mindestens einen Objektes insbesondere mit oder ohne dem Lagerbereich anzuzeigen. Für den Benutzer ist dies vorteilhaft, da er aufgrund dieser Informationen wesentliche Informationen erhält, die ihm zum einen eine genauere Versuchsplanung erlauben - bevor er einen Versuch durchführt weiß er, dass Stellfläche verfügbar ist; zum anderen beeinflusst die Positionsänderung von Proben, insbesondere in den ersten Stunden nach der Aussaat von adhärenten Zellen, negativ deren Anhaftung; es bildet sich dann kein gleichmäßiger Zellrasen aus. Die erfindungsgemäße Bereitstellung der Informationen zu Positionsänderungen bzw. deren Häufigkeit erlaubt es dem Benutzer Ursachen für ein ungleichmäßiges Zellwachstum zu ermitteln und diese so bei zukünftigen Versuchen zu berücksichtigen.

Eine gerätegesteuerte Behandlung des Inkubators ist vorzugsweise eine programmgesteuerte Behandlung, also eine durch ein Programm gesteuerte Behandlung. Unter einer programmgesteuerten Behandlung eines Objektes ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mittels der digitalen Datenverarbeitungseinrichtung, die insbesondere Bestandteil der Steuereinrichtung ist. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich. Eine gerätegesteuerte Behandlung des Inkubators kann insbesondere in Abhängigkeit von zuvor erfassten Bilddaten erfolgen. Die Bilderfassung durch das Bilderfassungssystem ist insbesondere eine programmgesteuerte Behandlung, nämlich Ablichtung des Lagerbereichs bzw. Objekts.

Die Datenspeichereinrichtung weist vorzugsweise mindestens einen Datenspeicher auf, der insbesondere ein flüchtiger oder ein nicht-flüchtiger Datenspeicher sein kann. Die vom Inkubator erfassten oder empfangenen Daten sind auf diesem mindestens einen Datenspeicher speicherbar, insbesondere in mindestens einer Datenbank, die in mindestens einem Datenspeicher gespeichert sein kann. Zu diesen Daten gehören insbesondere mindestens eine oder alle der folgenden Datenarten: Bilddaten, Standbilddaten, Videobilddaten, Objektdaten, kombinierte Bilddaten, erste und zweite Bilddaten, Identifikationsdaten, ID-Positionsdaten, Benutzeridentifikationsdaten, benutzerbezogene ID-Positionsdaten, Objektsidentifikationsdaten, Bewegungshistoriedaten, klassenbezogene ID-Positionsdaten, individualbezogene ID-Positionsdaten, Belegungszustandsdaten. Die Datenspeichereinrichtung ist vorzugsweise Bestandteil des Inkubators, also insbesondere in einem Gehäuse des Inkubators angeordnet. Sie kann aber auch Bestandteil eines externen Datenverarbeitungsgeräts sein, mit dem der Inkubator bzw. dessen Datenverarbeitungseinrichtung kommuniziert.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Unter einem Programm bzw. Programmcode bzw. Computerprogrammcode wird insbesondere ein ausführbares Computerprogramm verstanden. Dieses liegt in einem Datenspeicher oder auf einem Datenspeichermedium vor. Ein Programm ist eine Folge von Anweisungen, insbesondere bestehend aus Deklarationen und Instruktionen, um auf einer digitalen Datenverarbeitungseinrichtung eine bestimmte Funktionalität, Aufgaben- oder Problemstellung bearbeiten und/oder lösen zu können. Ein Programm liegt in der Regel als Software vor, die mit einer Datenverarbeitungseinrichtung verwendet wird. Das Programm kann insbesondere als Firmware vorliegen, im Fall der vorliegenden Erfindung insbesondere als Firmware der Steuereinrichtung des Inkubators oder des Systems. Das Programm liegt meist auf einem Datenträger als ausführbare Programmdatei, häufig im sogenannten Maschinencode vor, die zur Ausführung in den Arbeitsspeicher des Rechners der Datenverarbeitungseinrichtung geladen wird. Das Programm wird als Abfolge von Maschinen-, d. h. Prozessorbefehlen von dem/den Prozessoren des Computers verarbeitet und damit ausgeführt. Unter "Computerprogramm" wird insbesondere auch der Quelltext des Programms verstanden, aus dem im Verlauf der Steuerung des Laborgerätes der ausführbare Code entstehen kann.

Eine Benutzerschnittstelleneinrichtung kann Bestandteil eines Inkubators sein, oder ein Modul. Eine Benutzerschnittstelleneinrichtung weist jeweils vorzugsweise auf: eine Steuereinrichtung für die Benutzerschnittstelleneinrichtung; eine Kommunikationseinrichtung zur Herstellung einer Datenverbindung mit einem Laborgerät, insbesondere einem Inkubator, über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer, insbesondere ein berührungsempfindliches Display. Dabei ist die Steuereinrichtung der Benutzerschnittstelleneinrichtung vorzugsweise dazu eingerichtet, über die Datenverbindung Daten mit der Steuereinrichtung des Inkubators auszutauschen.

Ein Objekt ist insbesondere ein Zellkulturbehälter. Ein Zellkulturbehälter ist insbesondere transparent. Er ist insbesondere aus Kunststoff gefertigt, insbesondere PE oder PS gefertigt und weist insbesondere eine planare Bodenplatte auf, welche die Wachstumsfläche der Zellen bildet. Diese kann eine Oberflächenbehandlung zur Förderung der Adhärenz von Zellen aufweisen. Der Zellkulturbehälter kann mit einer PE-Kappe oder Gasaustauschkappe, insbesondere einem Deckel mit optional enthaltenem Filter, verschließbar sein bzw. versehen sein. Der Zellkulturbehälter ist insbesondere stapelbar. Geeignet ist insbesondere eine Eppendorf Zellkulturflasche. Das Objekt kann ein Stapel von Zellkulturbehältern sein, insbesondere ein Stapel von Petrischalen oder von Zellkulturflaschen.

Vorzugsweise ist die Datenverarbeitungseinrichtung dazu programmiert, aus einem oder mehreren Bildern (zeitabhängige) Änderungen des Erscheinungsbildes (oder das Erscheinungsbild) der Objekte, insbesondere zwischen längeren Zeitabständen von Minuten, Stunden oder Tagen, zu erfassen. Auf diese Weise können Farbänderungen des Zellkulturmediums bzw. Farben in einem Zellkulturbehälter oder Strukturen, z.B. Tropfen, an einer Zellkulturbehälterwand ermittelt werden. Solche Farben, Farbänderungen oder Strukturen können auf Probleme der jeweiligen Zellkultur hinweisen, z.B. auf einen Nährstoffmangel, pH-WertVeränderungen, oder auf Schimmel, oder auf andere Kontaminationen. Vorzugsweise ist die Datenverarbeitungseinrichtung dazu programmiert, abhängig von der Detektion des Erscheinungsbildes eines Zellkulturbehälters bzw. dieser Änderungen des Erscheinungsbildes des Zellkulturbehälters eine Information über eine Benutzerschnittstelle an den Benutzer oder Betriebspersonal auszugeben und/oder die Daten über diese Detektion (insbesondere : was wurde wann detektiert) in einem Datenspeicher zu speichern und abrufbar vorzuhalten.

Bildverarbeitende Objektverfolgungstechniken sind grundsätzlich bekannt, zum Beispiel beim Einsatz in Drohnen oder Fahrassistenzsystemen zur Fahrzeug- oder Personenverfolgung. Die Objektverfolgung (englisch: "object tracking") basiert auf der Bildverarbeitung bzw. Bildauswertung von Videobilddaten. Solche Verfahren der Objektverfolgung lassen sich mit relativ einfachen Mitteln wie geeigneten Kameras und Bildverarbeitungsalgorithmen umsetzten. Die theoretischen Grundlagen und deren Nutzung zur praktischen Realisierung von Objektverfolgungstechniken sind bekannt (z.B.: "Fundamentals of Object Tracking", S. Challa et al., Cambridge University Press, 2011). Auch sofort verwendbare Bildverarbeitungsalgorithmen zur Objektverfolgung sind frei verfügbar (OpenCV.org) und gut dokumentiert. OpenCV (englische Abk. für Open Computer Vision) ist eine freie Programmbibliothek (BSD Lizenz) mit Algorithmen für die Bildverarbeitung und Computer Vision. Die Programmbibliothek OpenCV beinhaltet auch Funktionen zur Verfolgung mehrerer Objekte in Echtzeit. Die Anwendung von Objektverfolgung in Inkubatoren wurde bislang nicht publiziert und stellt eine Innovation dar.

Eine typische Arbeitsweise von bildverarbeitender Objektverfolgung, die vorzugsweise auch im Objektverfolgungssystem gemäß vorliegender Erfindung zum Einsatz kommt, basiert auf der Auswertung der zeitlichen Abfolge von Bildern. Ein typischer Programmcode zur Objektverfolgung verwendet eine "Bounding Box" als Ausgabeformat, um ein Objekt in einem Bild zu identifizieren, dessen Kollisionsgrenzen festzulegen und insbesondere zu lokalisieren. In der digitalen Bildverarbeitung bezeichnet die "Bounding Box" die Koordinaten des rechteckigen Rahmens, der ein im digitalen Bild gezeigtes Objekt größtenteils oder vollständig umschließt. Durch die Verwendung von Bounding Boxes bei der Objektverfolgung wird diese effizienter, da die Bildauswertung mittels eines solchen numerischen Hilfsmittels, insbesondere im Vergleich mit Algorithmen der Umrisserkennung von Objekten weniger Rechenschritte und damit eine geringere Rechenleistung erfordert. Zudem lassen sich die entsprechenden Algorithmen mithilfe von spezialisierten Grafikprozessoren (GPUs) effizient und kostengünstig ausführen. Geeignete Programmierschnittstellen (APIs) zur Objektverfolgung mittels Bounding Boxes stehen in der Programmbibliothek OpenCV unter den Bezeichnungen BOOSTING, CSRT, GOTURN, KCF, MEDIANFLOW, MOSSE, MIL, TLD zur Verfügung. Entsprechend stehen in OpenCV Programmbibliotheken ("MultiTracker") für das zeitgleiche Verfolgen von mehreren Objekten ("multiple object tracking") zur Verfügung. Als Alternative dazu sind Deep Learning Algorithmen zum Multi-Object Tracking (MOT) nach dem "tracking-by-detection" Prinzip bekannt.

Es ist aber auch möglich und bevorzugt, dass zur Objektverfolgung eine Ermittlung der Kontur des zu verfolgenden Objektes im Bild durchgeführt wird, und insbesondere der Trennung von Objekt (Vordergrund) und Hintergrund durch Hintergrundsubtraktion.

Das Leistungspotential eines Objektverfolgungssystems beruht einerseits auf einem zuverlässigen automatischen Identifizieren eines Objekts im Inkubator in verschiedenen typischen Nutzungsszenarien eines Inkubators, die nachfolgend beschrieben werden. Andererseits ist der Ansatz effizient, da auf Seiten des Objekts keine besonderen Anpassungen erforderlich sind. Insbesondere muss das Objekt keine passiven (Code, Beschriftung) oder aktiven (z.B. einen Sender) Identifikationshilfsmittel beinhalten. Es lassen sich vielmehr die üblichen Objekte (Zellkulturbehälter, Geräte etc.) mit dem Inkubator verwenden, insbesondere unabhängig von Hersteller und äußerlicher Erscheinung. Insbesondere ist der erfindungsgemäße Inkubator in der Lage, Objekte mit vollkommen identischem Aussehen durch die Verfolgung zu unterscheiden.

Mögliche Szenarien bei der Änderung der Belegung in einem Inkubator sind:
I. Einstellen neuer Objekte
II. Entnahme von Objekten
III. Tür wird geöffnet und es werden Objekte nur verschoben, ohne eines zu entnehmen oder neu einzustellen.

### Subkonditionen:

i) Objekt/e werden verschoben
ii) Objekt/e werden nicht verschoben
iii) Mehrere Objekte werden eingestellt/entnommen (Reihenfolge).

Annahme: alle Zellkulturbehälter sehen äußerlich gleich aus. Die der Entwicklung der Erfindung zugrunde liegende Frage war insbesondere, welche bildbasierten Methoden in Frage kommen, insbesondere ob Standbilder ausreichen, um in üblichen Nutzungsszenarien eines Inkubators eine Objektidentifikation zu ermöglichen.

Es wird für das Szenario I. (neues Objekt soll in die Inkubatorkammer eingesetzt werden) zunächst angenommen, dass es vor der Öffnung der Inkubatortüre ein aktuelles, von einer im Inkubator platzierten Kamera aufgenommenes Standbild des Lagerbereichs in der Inkubatorkammer gibt, welches das neue Objekt noch nicht zeigt.

Wird das neue Objekt ohne Verschieben der Bestandsobjekte (bereits im Lagerbereich angeordnete und lokalisierte Objekte) in die Inkubatorkammer eingestellt (Fall I.ii)), lässt sich das neue Objekt (problemlos eindeutig) über das nächste Standbild (nach Schließen der Inkubatortüre) identifizieren. Eine Objektverfolgung ist für den Fall I.ii) nicht notwendig. Entsprechendes gilt für II.ii): im Fall der Entnahme eines Objektes ist dessen Identifizierung eindeutig möglich aus der Evaluierung der Standbilder vor und nach Türöffnung.

Wird das neue Objekt eingestellt und werden dabei Bestandsobjekte verschoben (Fall I.i)), lässt sich das neue Objekt nicht eindeutig über das nächste Standbild identifizieren. Standortinformationen über die bereits registrierten Bestandsobjekte gehen verloren. Dasselbe gilt für die Entnahme eines Objektes und dabei erfolgendes Verschieben der Bestandsobjekte (Fall II.i)). Für das Verschieben (Kondition i)) kommt das Konzept der Objektverfolgung zu tragen.

Werden mehrere Objekte (Fall iii)) unter der Bedingung i), d.h. ohne Verschieben der Bestandsobjekte, eingestellt, lassen sich zwar die neuen Objekte per vorher-nachher-Standbilder einfach identifizieren, es geht aber die Information über die Reihenfolge des Einstellens verloren. Will man diese Information erhalten, benötigt man die Objektverfolgung. Entsprechendes gilt für den Fall der Entnahme mehrerer Objekte im Fall i) + iii).

Da im Betrieb eines Inkubators ein Verschieben von Bestandsobjekten eher die Regel als die Ausnahme ist, reicht in diesem Fall eine Auswertung der vorher-nachher-Standbilder des Lagerbereichs nicht aus.

Eine Frage bei der Entwicklung eines Objektverfolgungssystems in einem Inkubator ist insbesondere: Wann wird ein Objekt identifiziert, d.h. zu welchem Zeitpunkt bzw. bei welchem Ereignis werden dem Objekt eine Identifikationsdaten zugeordnet? In den meisten Anwendungsszenarien (abgesehen von einem Fall wie iii)+i), bei dem es ggf. doch auf die Erfassung der Reihenfolge des Einstellens von Objekten ankommt) reicht es aus, diese Identifikationsdaten zu erheben, wenn die neuen Objekte eingestellt wurden und dabei eventuell Bestandsobjekte verschoben wurden. Denn das Verschieben der bereits registrierten Bestandsobjekte erfolgt unter der Maßnahme der Objektverfolgung. Im nächsten Standbild, also insbesondere bei geschlossener Inkubatortüre, kann dann die Registrierung der neuen Objekte anhand des Standbildes erfolgen. Falls doch die Erfassung der Reihenfolge gewünscht ist, kann in dem Moment, in dem ein Objekt erstmals in den Sichtbereich der Kamera eintritt, also erstmals in einem von der Kamera aufgenommenen Bild (ein Startbild, das hier ein Videobild ist) auftritt, wird dieses registriert werden, also dem Objekt eine ID-Nummer zugeordnet, und es wird dieses Objekt verfolgt bis zu seiner Endposition, das eventuelle Verschieben von Bestandsobjekten oder deren Entnahme wird dabei vorzugsweise auch verfolgt.

In einem anderen praxisrelevanten Szenario wird von einer Belegung des Lagerbereichs (oder mehrerer Lagerbereiche) mit einem oder mehreren Objekten (Bestandsobjekten) ausgegangen, die anhand eines ersten Startbildes (in diesem Fall z.B. ein Standbild) registriert werden. Es muss hier nur die eventuell stattfindende Verschiebung dieser Bestandsobjekte verfolgt werden. Die Bewegung neu einzustellender Objekte muss beim Vorgang des Einstellens hier nicht verfolgt werden, denn deren Registrierung kann wieder im nächsten Standbild erfolgen. Die entsprechende Präsenz dieser neuen Objekte in den Videodaten kann somit ignoriert werden. In diesem Szenario geht die Information über die Reihenfolge des Einstellens mehrerer Objekte während einer Türöffnung verloren, aber diese Information wird auch nicht zwingend benötigt.

Die Erfindung schlägt demnach für eine bevorzugten Ausführungsform vor, eine Objektverfolgung zu implementieren, um die korrekte Lokalisation von Objekten, je nach Bedarf, in verschiedenen oder allen Situationen zu gewährleisten.

Die Datenverarbeitungseinrichtung ist insbesondere dazu programmiert, dem in den Innenraum eingebrachten mindestens einen Objekt Identifikationsdaten zuzuordnen. Dies bedeutet insbesondere, dass in den Bilddaten (Standbilder oder Videodaten) der Kamera ein neues Objekt detektiert wird. Insbesondere wird ein neues Objekt dann detektiert, wenn dieses in den Kamerasichtbereich der Kamera von außen hineinbewegt wird. Mit dem Detektieren des Objektes können diesem einerseits Identifikationsdaten zugeordnet werden, andererseits auch Positionsdaten. Die Positionsdaten, insbesondere Start- und Endposition eines Objektes, werden insbesondere mit Bezug auf ein internes Koordinatensystem bestimmt, anhand dem auch die Lage des mindestens einen Lagerbereichs und damit auch die Lage des mindestens einen Objektes zu dem mindestens einen Lagerbereich definiert wird. Diese Lageinformationen sind insbesondere dafür von Bedeutung, wenn dem Benutzer auf einem Display die Position des mindestens einen Objektes in dem mindestens einen Lagerbereich bzw. in der Inkubatorkammer grafisch veranschaulicht werden soll.

Identifikationsdaten können eine Identifikationsnummer sein oder diese beinhalten, und/oder können einen Identifikationscode aus beliebigen Zeichen bzw. Informationen beinhalten. Diese Identifikationsdaten können vorbestimmt sein, zufallsgeneriert sein, oder von einem Benutzer vorgegeben sein, insbesondere solange sie geeignet sind, das neu in die Inkubatorkammer eingestellte Objekt gegenüber den Identifikationsdaten der anderen Bestandsobjekte eindeutig zu unterscheiden. Die Identifikationsdaten können auch vorbestimmt sein und lediglich ausgewählt werden. Letzteren Fall umfasst der Begriff "zuordnen" ebenso wie die Neuerstellung der Identifikationsdaten.

Die Datenverarbeitungseinrichtung ist insbesondere dazu programmiert, die Startposition des mindestens einen Objektes aus dem Startbild des Lagerbereichs zu ermitteln. Das Startbild ist vorzugsweise ein Standbild, das in einem Standbildmodus der Kamera aufgenommen wird. Es kann auch ein aus Videodaten gewonnenes Einzelbild sein, insbesondere ein Videoframe. Die Datenverarbeitungseinrichtung ist insbesondere dazu programmiert, eine einhüllende Linienfigur, vorzugsweise Rechteck, oder einen einhüllenden Körper, oder insbesondere eine Bounding Box des Objektes, oder eine Außenkontur des Objektes, in dem Startbild zu festzulegen und, insbesondere, das Objekt als den von der einhüllenden Linienfigur, insbesondere von der Bounding Box oder einer Außenkontur zu definieren.

Die Datenverarbeitungseinrichtung ist insbesondere dazu programmiert, die Positionsänderungen des mindestens einen Objektes durch Auswertung der Videodaten zu ermitteln. Die Datenverarbeitungseinrichtung ist insbesondere dazu programmiert, die Bewegung des im Startbild mittels der Bounding Box definierten Objektes zu verfolgen. Die Datenverarbeitungseinrichtung ist insbesondere dazu programmiert, die Bewegung des im Startbild mittels der Bounding Box definierten Bildbereichs, der das Objekt enthält, zu erkennen, indem die Positionsänderungen dieses Bildbereichs von Frame zu Frame ermittelt werden. Dabei kann insbesondere eine Verfolgung der Bounding Box verwendet werden, um den sich aufgrund der Objektbewegung positionsverändernden Bildbereich zu ermitteln. Videodaten enthalten insbesondere Informationen, mittels denen sich die das Video kennzeichnenden Einzelbilder ("frames", die im Falle der Darstellung des Videos mit bestimmter Anzahl pro Zeit also "framerate" dargestellt werden) rekonstruieren lassen. Im Fall nicht komprimierter Videodaten können letztere auch die komplette Sequenz von Bilddaten enthalten, wobei ein "Satz" Bilddaten jeweils ein Einzelbild repräsentiert. Im Fall komprimierter Bilddaten werden eventuell auch/nur zeitliche Veränderungen von Pixeln des Kamerabilds erfasst.

Die Datenverarbeitungseinrichtung ist insbesondere dazu programmiert, aus dem Startbild die Startposition des Objektes im Lagerbereich zu ermitteln. Die Startposition kann insbesondere dadurch bestimmt sein, dass das in einem ersten Frame einer Bilderserie zuvor unbewegte Objekt im nachfolgenden Frame eine Positionsänderung des Objekts zeigt. Der erste Frame, in dem das Objekt gegenüber den vorangegangenen Frames eine Positionsänderung aufweist, kann als das Startbild definiert werden. Da die Bewegung des Objektes zu einem Zeitpunkt T1 beginnt und zu einem Zeitpunkt T2 endet, kann ein Bild (Standbild oder Videobild, auch ein Bild gewonnen aus superpositionierten Bildern), das vor dem Zeitpunkt T1 erfasst wird, als Startbild herangezogen werden, aus dem die Startposition des mindestens einen Objektes festgelegt wird.

Die Datenverarbeitungseinrichtung ist insbesondere dazu programmiert, aus den Positionsänderungen die Endposition des mindestens einen Objektes im Endbild des Lagerbereichs zu ermitteln. Die Endposition kann insbesondere dadurch bestimmt sein, dass von Frame zu Frame keine Positionsänderungen des Objekts mehr bestimmt werden. Der erste Frame, in dem das Objekt gegenüber den vorangegangenen Frames keine Positionsänderung mehr aufweist, kann als das Endbild definiert werden. Da die Bewegung des Objektes zu einem Zeitpunkt T1 beginnt und zu einem Zeitpunkt T2 endet, kann ein Bild (Standbild oder Videobild, auch ein Bild gewonnen aus superpositionierten Bildern), das ab dem Zeitpunkt T2 erfasst wird, als Endbild herangezogen werden, aus dem die Endposition des mindestens einen Objektes festgelegt wird. Die Endposition kann insbesondere durch den Zeitpunkt bestimmt sein, zu dem mittels des Türsensors ein Schließen der Inkubatortüre erfasst wird. Die Endposition kann insbesondere dadurch bestimmt sein, dass ein in den Bildbereich hineinführender Arm oder eine Hand des Benutzers nicht mehr erfasst wird. Beispielsweise kann ein Bild dahingehend ausgewertet werden, ob ein im Bildrandbereich gelegener, z.B. streifenförmiger, Ausschnitt einem Referenzzustand entspricht, in dem ein Referenzausschnitt der Inkubatorkammer bzw. des Inkubators vollständig sichtbar ist. Ist dies nicht der Fall, kann geschlussfolgert werden, dass ein Benutzer immer noch im Inneren des Inkubators hantiert und das Objekt oder mehrere Objekte immer noch bewegt werden, so dass insbesondere die Videobilderfassung und -auswertung fortzusetzen ist. Die Endposition des Objektes kann als die Position verstanden werden, an der das Objekt nach vorangegangen Positionsänderungen keine Positionsänderung mehr zeigt, kann also durch das Ende der Bewegung des Objektes bestimmt sein. Alternativ oder zusätzlich kann die Endposition des Objektes so definiert sein, dass die Position, die das Objekt inne hat, wenn das Schließen einer Inkubatortüre mittels Türsensor erfasst wird, die Endposition ist. Im Resultat wird die Endposition in den meisten Fällen dieselbe sein.

Die Datenverarbeitungseinrichtung ist vorzugsweise dazu programmiert, die Erfassung des Startbilds und/oder von Videodaten mittels der Kamera zu starten, wenn ein Sensor eine am Inkubator erfolgende Aktivität detektiert. Der Sensor kann ein Bewegungssensor sein, der eine Bewegung in einem außerhalb des Inkubators gelegenen Detektionsbereich detektiert. Der Sensor kann ein Berührungssensor sein, der eine von einem Benutzer durchgeführte Berührung des Inkubators, z.B. eines Türgriffs des Inkubators detektiert. Der Sensor kann ein Türöffnungssensor sein, der insbesondere ein Öffnen einer Inkubatortüre, insbesondere eines Außentüre des Inkubators, erfasst. Der Sensor kann eine Außenkamera des Inkubators sein, die mittels Bildauswertung eine Bewegung und/oder eine Person im Sichtbereich der Kamera erfasst. Der Sensor kann ein Annäherungssensor sein, der, z.B. durch Detektion der Änderung eines elektrischen Feldes, die Annäherung einer Person an den Inkubator erfasst.

Die Datenverarbeitungseinrichtung ist vorzugsweise dazu programmiert, die Erfassung des Endbilds zu starten und/oder die Erfassung von Videodaten zu stoppen, wenn ein Sensor eine am Inkubator erfolgende Aktivität detektiert. Der Sensor kann ein Türöffnungssensor sein, der insbesondere ein Schließen einer Inkubatortüre, insbesondere eines Außentüre des Inkubators, erfasst. Der Sensor kann eine Außenkamera des Inkubators sein, die mittels Bildauswertung die Beendigung einer Bewegung und/oder das Verschwinden einer Person im Sichtbereich der Kamera erfasst. Der Sensor kann ein Annäherungssensor sein, der, z.B. durch Detektion der Änderung eines elektrischen Feldes, das Entfernen einer Person vom Inkubator erfasst.

Die Datenverarbeitungseinrichtung ist insbesondere dazu programmiert, die Erfassung von Videodaten mittels der Kamera initiiert vom mittels Türsensor detektierten Öffnen einer Inkubatortüre zu starten. Alternativ oder zusätzlich kann ein Initialereignissensor, insbesondere ein Bewegungssensor, oder Annäherungssensor, oder optischer Sensor/Empfänger (z.B. Lichtschranke), oder Mikrofon, oder Beschleunigungssensor in der Inkubatortüre, im Inkubator angeordnet sein, mittels dem die Annäherung eines Objektes an die Inkubatorkammer oder eines anderen Initialereignisses erfassbar ist; Auslöser für die Initiierung der Kamera und Videodaten kann auch eine Codeeingabe an einem Türschloss des Inkubators sein, welche insbesondere von der Datenverarbeitungseinrichtung ohne Verwendung von Messergebnissen von einem der genannten Sensoren durchgeführt werden kann; die Datenverarbeitungseinrichtung kann programmiert sein, basierend auf den Daten eines solchen Sensors die Erfassung von Videodaten zu starten. Die Datenverarbeitungseinrichtung kann programmiert sein, mit Vorliegen der Videodaten und/oder mit Vorliegen eines Standbildes die Suche nach einem neuen Objekt in den mittels der Videodaten verfügbaren Bilder (Frames) oder dem Standbild zu beginnen. Alternativ kann die Erfassung von Videodaten starten, sobald ein Benutzer am Inkubator identifiziert wurde, oder bei einem anderen vorgegebenen Ereignis. Auch eine permanente Videodatenerfassung ist möglich. Die Datenverarbeitungseinrichtung ist insbesondere dazu programmiert, die Erfassung von Videobilddaten mit der Erfassung des Endbildes bzw. dem Registrieren der Abwesenheit einer Hand/eines Arms im Kamerasichtbereich zu beenden, oder basierend auf den Ergebnissen eines der genannten Sensoren (Türsensor, Bewegungssensor etc.) zu beenden.

Die Datenverarbeitungseinrichtung ist insbesondere dazu programmiert, dem mindestens einen Objekt im Lagerbereich jeweils Identifikationsdaten zuzuordnen und die Position des mindestens einen Objektes jeweils als ID-Positionsdaten zu ermitteln und in dem Datenspeicher zu speichern. Die Datenverarbeitungseinrichtung ist insbesondere dazu programmiert, die Endposition des mindestens einen Objektes im Lagerbereich in Abhängigkeit von den Identifikationsdaten des mindestens einen Objektes als ID-Positionsdaten in dem Datenspeicher zu speichern. Mit diesem Schritt "kennt" der Inkubator "das Objekt" und dessen Position. Zusammen mit anderen Daten kann er diese Daten nun einem Benutzer ausgeben, insbesondere auf einem Display des Inkubators anzeigen. Zusammen mit Daten über den Eigentümer (Definition Eigentümer: der Benutzer, der das Objekt in die Inkubatorkammer eingestellt hat) des Objektes oder einen Benutzer des Objektes (z.B. ein Benutzer, der ein Bestandsobjekt eines anderen Benutzers bewegt hat) kann der Inkubator diese Daten-sätze in Abhängigkeit von den Identifikationsdaten des Objektes speichern und sammeln. Die Identifikationsdaten, auf Basis derer die Positionsänderungen detektiert wurden, müssen nicht identisch sein mit den Identifikationsdaten, die als ID-Positionsdaten gespeichert werden; relevant ist, dass die abgespeicherten Identifikationsdaten geeignet sind, das mindestens eine Objekt eindeutig von anderen Objekten bzw. Bestandsobjekten zu unterscheiden. Der ID-Code kann also theoretisch während der Bildverarbeitung wechseln.

Die Zuordnung eines Eigentümers zu einem Objekt kann auf unterschiedliche Weise erfolgen. Vorzugsweise ist die Datenverarbeitungseinrichtung dazu programmiert, den das Objekt in den Inkubator einstellenden Benutzer zu registrieren oder zu identifizieren, diesem Benutzer einen Benutzeridentifikationscode zuzuordnen, und benutzerbezogenen ID-Positionsdaten des Objektes zu speichern. Für eine Registrierung kann eine Biometrieerkennung, insbesondere Gesichtserkennung, Spracherkennung, und/oder Stimmerkennung, des Benutzers durchgeführt werden, insbesondere mittels einer Außenkamera, eines Retinascanners oder eines Fingerabdrucksensors des Inkubators. Die entsprechenden registrierten Biometrieerkennungsdaten, insbesondere Gesichtserkennungsdaten des Benutzers können in der Datenspeichereinrichtung des Inkubators, oder in einer externen Datenspeichereinrichtung gespeichert werden. Die Identifizierung des Benutzers kann anhand eines Abgleichs von erfassten Biometrieerkennungsdaten mit bereits registrierten Biometrieerkennungsdaten erfolgen. Alternativ zu einer Biometrieerkennung kann es einem Benutzer auch ermöglicht werden, vor, während oder nach der Durchführung der Objektregistrierung bzw. nach der Ermittlung der Endposition eines verfolgten Objektes einen Benutzeridentifikationsdaten über eine Benutzerschnittstelleneinrichtung einzugeben. Die Benutzerschnittstelleneinrichtung kann eine Tastatur, ein Touchscreen sein und kann Teil des Inkubators oder eines Externgeräts sein, oder eine Benutzername/- kennung kann mittels Spracheingabe eingebbar sein.

Ein Vorteil der Objektverfolgung ist, dass diese grundsätzlich ohne Kenntnis von Individual- oder Klassen-Merkmalen des zu verfolgenden Objektes durchgeführt werden kann. Sie kann aber auch mit Methoden zur Objekterkennung (und -wiedererkennung) und/oder der Objektklassenerkennung bzw. Klassenwiedererkennung kombiniert werden. Dies ist insbesondere hilfreich, wenn mehrere Objekte mittels Objektverfolgungssystem parallel verfolgt werden.

Eine **Objekterkennung** kann insbesondere als Objektindividualerkennung und/oder als Objektklassenerkennung ausgestaltet sein. Die theoretischen Grundlagen und deren Umsetzung zur praktischen Anwendung in Objekterkennungstechnologien sind bekannt (z.B.: "Deep Learning in Object Detection and Recognition", X. Jiang et al., Springer Singapore, 2019). Algorithmen zur Objekterkennung in Bildern sind allgemein bekannt und verfügbar, z.B. als Bestandteil von OpenCV (for example OpenCV 3.3, deep neural network (dnn) module).

Eine Objektindividualerkennung basiert auf der Erkennung objektindividueller Merkmale (Objektindividualmerkmale), anhand der ein Wiedererkennen des individuellen Objektes und ein Unterscheiden gegenüber anderen individuellen Objekten möglich ist. Beispielsweise kann ein Zellkulturbehälter, z.B. ein Einwegprodukt, nachträglich aufgebrachte Individualmerkmale aufweisen, z.B. einen Barcode oder QR-Code. Er kann aber auch über jegliche Merkmale identifizierbar sein, die eine Unterscheidung ermöglichen: z.B. eine Beschriftung, einen unterschiedlichen Inhalt, ein Mikrokratzermuster an der Behälterfläche etc..

Eine Objektklassenerkennung beruht auf der Kenntnis von Objektklassenmerkmalen, die bei der Objektprüfung abgeglichen werden, um dem Objekt eine Klasse zuzuordnen. Beispielsweise kann mit einer Objektklassenerkennung erkannt werden, ob ein Objekt eine bestimmte Art von Zellkulturflasche, eine bestimmte Art von Petrischale oder eine bestimmte Art von Mikrotiterplatte ist, eventuell mit Berücksichtigung weiterer Klassenmerkmale, z.B. Hersteller, Herstellungsjahr, Ausführung, etc..

Der Inkubator weist vorzugsweise ein Objekterkennungssystem auf. Das Objektverfolgungssystem ist vorzugsweise zusätzlich zur Objekterkennung eingerichtet.

Im Falle der Objektindividualerkennung ist eine Datenverarbeitungseinrichtung des Objekterkennungssystems oder des Objektverfolgungssystems vorzugsweise programmiert, an einem Standbild, dem Startbild, den Videodaten und/oder dem Endbild Individualmerkmale mindestens einen Objektes zu erkennen, und b) diese Individualmerkmale des Objektes in Form von Objektindividualdaten zu speichern, insbesondere in Abhängigkeit von Identifikationsdaten. Vorzugsweise ist die Datenverarbeitungseinrichtung programmiert, Objektindividualmerkmale des mindestens einen Objektes aus dem Startbild, den Videodaten und/oder dem Endbild zu entnehmen, die Objektindividualmerkmale mit einer Objektindividualdatenbank abzugleichen und, falls die Objektindividualmerkmale in der Objektindividualdatenbank mit einer Objektindividualkennung verbunden sind: die Objektindividualkennung des mindestens einen Objektes zu identifizieren; oder, falls die Objektindividualmerkmale in der Objektindividualdatenbank nicht mit einer Objektindividualkennung verbunden sind: dem mindestens einen Objekt eine Objektindividualkennung zuzuordnen und in der Objektindividualdatenbank zu speichern, und/oder den ID-Positionsdaten des mindestens einen Objektes die erkannte Objektindividualkennung zuzuordnen und als individualbezogene ID-Positionsdaten zu speichern. Eine Objektindividualkennung ist vorzugsweise unterschiedlich zu dessen Identifikationsdaten; die Objektindividualkennung kann aber auch vorzugsweise gleich zu dessen Identifikationsdaten sein.

Im Falle der Objektklassenerkennung ist eine Datenverarbeitungseinrichtung des Objekterkennungssystems oder des Objektverfolgungssystems vorzugsweise programmiert, a) in einem Standbild, dem Startbild, den Videodaten und/oder dem Endbild Klassenmerkmale des mindestens einen Objektes zu erkennen, und b) diese Klassenmerkmale des Objektes in Form von Objektklassendaten zu speichern, insbesondere in Abhängigkeit von Identifikationsdaten. Vorzugsweise ist die Datenverarbeitungseinrichtung programmiert, Objektklassenmerkmale des mindestens einen Objektes in einem Standbild, dem Startbild, den Videodaten und/oder dem Endbild zu erkennen, die Objektklassenmerkmale mit einer Objektklassendatenbank (die insbesondere vorbekannte Korrelationen zwischen der Objektklasse und Objektklassenmerkmalen enthält) abzugleichen und die Objektklasse des mindestens einen Objektes zu erkennen, und insbesondere den ID-Positionsdaten des mindestens einen Objektes die erkannte Objektklasse als Objektklassendaten zuzuordnen und insbesondere als klassenbezogene ID-Positionsdaten zu speichern.

Vorzugsweise weist der Inkubator eine Benutzeridentifikationseinrichtung auf, mittels der ein den Inkubator benutzender Benutzer in Form von Benutzeridentifikationsdaten identifizierbar ist. Vorzugsweise ist eine Datenverarbeitungseinrichtung des Inkubators dazu programmiert, einen den Inkubator benutzenden Benutzer mittels der Benutzeridentifikationseinrichtung zu identifizieren und ihm Benutzeridentifikationsdaten zuzuordnen und Identifikationsdaten und/oder ID-Positionsdaten in Abhängigkeit von den Benutzeridentifikationsdaten als benutzerbezogene Identifikationsdaten und/oder benutzerbezogene ID-Positionsdaten in dem Datenspeicher zu speichern.

Vorzugsweise weist die Benutzeridentifikationseinrichtung eine Außenkamera auf, und vorzugsweise ist die Benutzeridentifikationseinrichtung dazu eingerichtet, und/oder die Datenverarbeitungseinrichtung dazu programmiert, mittels der Außenkamera eine Gesichtserkennung durchzuführen, mittels der der Benutzer identifiziert wird. Vorzugsweise ist eine Benutzerdatenbank vorgesehen, die auf einem Datenspeicher gespeichert ist, der Teil des Inkubators, der Benutzeridentifikationseinrichtung oder des Objektverfolgungssystems sein kann, oder der in einer Datenaustauschverbindung mit der Benutzeridentifikationseinrichtung bzw. der Datenverarbeitungseinrichtung stehen kann, z.B. über ein Intranet oder das Internet. Algorithmen zur Gesichtserkennung in Bildern sind allgemein bekannt und verfügbar, z.B. als Bestandteil von OpenCV ("FaceRecognizer class").

Die Benutzerdatenbank kann eine Korrelation von Benutzeridentifikationsdaten und Benutzermerkmalsdaten enthalten, so dass der Benutzer bzw. dessen Benutzeridentifikationscode (Benutzeridentifikationsdaten) anhand der ermittelten oder eingelesenen Benutzermerkmalsdaten ermittelbar ist. Die Benutzermerkmalsdaten können Informationen über Gesichtsmerkmale der Benutzer enthalten, oder andere biometrische Daten, z.B. Fingerabdruckdaten oder Stimmenerkennungsdaten. Die Benutzerdatenbank kann eine Korrelation von Benutzeridentifikationsdaten und Benutzerkennungen enthalten, wobei die Benutzerkennung ein persönlicher Identifikationscode des Benutzers sein kann, z.B. eine mehrstellige Zeichenfolge, über deren Eingabe an einer Tastatur der Benutzerschnittstelleneinrichtung sich ein Benutzer identifizieren kann.

Die Außenkamera kann insbesondere an, oberhalb oder neben einer Inkubatortüre, insbesondere Außentüre am Inkubator angeordnet bzw. an diesem befestigt sein. Vorzugsweise ist die Außenkamera ein fester Bestandteil des Inkubators oder der Inkubatortüre. Sie kann aber auch über eine Signalverbindung, insbesondere über eine Datenaustauschverbindung, die kabelgebunden oder kabellos sein kann, mit der Benutzeridentifikationseinrichtung bzw. der Datenverarbeitungseinrichtung verbunden sein. Z.B. ist es möglich, die Außenkamera über ein flexibles Kabel mit dem Inkubator bzw. dessen Benutzeridentifikationseinrichtung bzw. der Datenverarbeitungseinrichtung zu verbinden, so dass die Kamera vom Benutzer frei am Inkubator platzierbar ist.

Vorzugsweise weist die Benutzeridentifikationseinrichtung eine Benutzerschnittstelleneinrichtung auf, mittels der Benutzeridentitätsdaten einlesbar sind. Die Benutzerschnittstelleneinrichtung kann eine Tastatur beinhalten, und/oder einen Touchscreen, und oder ein Mikrofon für eine Spracheingabe bzw. zur Realisierung einer Benutzeridentifikation mittels einer Spracherkennung. Die Benutzerschnittstelleneinrichtung kann zum Austausch von Daten mit einem externen datenverarbeitenden Gerät (nachfolgend auch als "Externgerät" bezeichnet) eingerichtet sein. Das Externgerät kann ein PC sein, ein Smartphone, ein Tabletcomputer, oder ein anderer portabler Computer mit Benutzerschnittstelle.

Das Externgerät kann Mittel aufweisen, um einen Benutzer zu identifizieren und/oder zu authentifizieren. Insbesondere aktuell erhältliche Smartphones beinhalten diverse Mittel zur Nutzerauthentifizierung, insbesondere zur Gesichtserkennung. Das Externgerät weist vorzugsweise eine Software auf, z.B. eine App, die dazu programmiert ist, einen Benutzer zu identifizieren und/oder zu authentifizieren, und insbesondere das Ergebnis dieses Vorgangs über die Benutzerschnittstelleneinrichtung an die Benutzeridentifikationseinrichtung des Inkubators zu übersenden. Da ein Externgerät auch oft eine eigene Kamera aufweist, anhand der eine Gesichtserkennung implementiert sein kann, oder einen Fingerabdrucksensor, oder sonstige Hardware zur Benutzeridentifikation und -authentifizierung, sind die entsprechenden Hardwarebestandteile im Fall der Verbindung des Inkubators mit dem Externgerät am Inkubator verzichtbar.

Die Benutzeridentifikationseinrichtung des Inkubators kann als Bestandteil einer Steuerungssoftware des Inkubators programmiert sein. Der Inkubator weist vorzugsweise eine Steuerungseinrichtung auf, die insbesondere eine Datenverarbeitungseinrichtung aufweisen kann, die insbesondere dazu programmiert sein kann, alle oder einige Funktionen der Benutzeridentifikationseinrichtung zu beinhalten, insbesondere den Datenaustausch mit dem Externgerät zu steuern.

Vorzugsweise weist die Benutzeridentifikationseinrichtung eine Benutzerschnittstelleneinrichtung auf, mittels der Benutzeridentitätsdaten auswählbar sind. Dazu kann insbesondere die Benutzeridentifikationseinrichtung ein Display bzw. einen Touchscreen aufweisen, über das eine Liste möglicher Benutzer, z.B. über die Angabe eines Namens oder Bildes des Benutzers, anzeigbar ist. Es können dann Eingabemittel vorgesehen sein, z.B. Tasten, eine Tastatur, Touchpad, der Touchscreen, über die der Benutzer die Auswahl aus der Liste vornehmen kann.

Die Benutzeridentifikationseinrichtung kann dazu programmiert sein, eine Benutzerauthentifizierung vorzunehmen, indem das genannte Einlesen der Benutzeridentitätsdaten oder die genannte Auswahl aus der Liste passwortgeschützt ist, so dass der Benutzer erst als identifiziert gilt, wenn die Authentifizierung erfolgreich war.

Vorzugsweise weist die Benutzeridentifikationseinrichtung ein Lesegerät zum Einlesen eines den Benutzer identifizierenden Codes auf, wobei das Lesegerät insbesondere ein RFID-Lesegerät, ein Barcode-Lesegerät, oder ein QR-Code-Lesegerät ist.

Die Benutzeridentifikationseinrichtung bzw. eine/deren Datenverarbeitungseinrichtung kann dazu programmiert sein, eine verriegelte Inkubatortüre in Abhängigkeit von der Benutzeridentifikation zu entriegeln und/oder zu verriegeln, insbesondere eine verriegelte Inkubatortüre zu entriegeln, wenn der Benutzer erfolgreich identifiziert wurde. Dies bedeutet in diesem Fall, dass der Benutzer auch für den Inkubator zugangsberechtigt ist. Es kann aber auch eine zusätzliche Zugangsrechteliste geben, anhand derer der Inkubator entscheidet, ob ein identifizierter Benutzer das Zugangsrecht besitzt oder, ggf. welche Art Zugangsrecht der identifizierte Benutzer besitzt. Das Zugangsrecht kann z.B. beschränkt sein auf bestimmte Zeiten, insbesondere Wochentage, Uhrzeiten, oder Berechtigungszeitabschnitte. Wenn der Inkubator mehrere Inkubatortüren aufweist, kann das Zugangsrecht vorsehen, dass der Benutzer nur für eine vorbestimmte Auswahl dieser Inkubatortüren das Zugangsrecht besitzt.

Vorzugsweise weist der Inkubator genau eine -oder auch mehrere- Inkubatortüre(n) zum Verschließen der Kammeröffnung auf. Die Inkubatortüre bildet im verschlossenen Zustand insbesondere einen Teil des Inkubatorgehäuses, dem das als thermischer Isolator der Inkubatorkammer des Inkubators dient. Die Inkubatortüre kann an ihrer Außenseite eine Benutzerschnittstelleneinrichtung aufweisen, insbesondere ein Display. Eine Datenverarbeitungseinrichtung des Inkubators bzw. der Benutzerschnittstelleneinrichtung kann dazu programmiert sein, ein von der Kamera des Inkubators aufgenommenes Bild des mindestens einen Lagerbereichs des Inkubators anzuzeigen.

Vorzugsweise weist der Inkubator einen Türsensor zum Erfassen des Öffnens bzw. des Schließens der Inkubatortüre auf. Vorzugsweise weist der Inkubator einen Bewegungssensor oder einen Annäherungssensor zum Erfassen einer sich dem Inkubator nähernden Person auf. Vorzugsweise ist die Datenverarbeitungseinrichtung dazu programmiert, die Überwachung des Innenraums des Inkubators, insbesondere die Generierung der Videodaten / Standbilddaten, in Abhängigkeit von der Detektion einer Türöffnung des Inkubators und/oder der Annäherung einer Person zu starten;
vorzugsweise ist die Datenverarbeitungseinrichtung dazu programmiert, die Überwachung des Innenraums des Inkubators, insbesondere die Generierung der Videodaten / Standbilddaten zu starten, insbesondere in Abhängigkeit von der Detektion einer Türöffnung durch einen Benutzer, der mittels einer Benutzeridentifikationseinrichtung identifiziert wurde;
vorzugsweise ist die Datenverarbeitungseinrichtung dazu programmiert, die Überwachung des Innenraums des Inkubators, insbesondere die Generierung der Videodaten, in Abhängigkeit von der Detektion einer Türschließung des Inkubators zu beenden;
vorzugsweise ist die Datenverarbeitungseinrichtung dazu programmiert, mittels der Informationen aus der Benutzeridentifikationseinrichtung und der Objektverfolgungseinrichtung festzustellen, durch welchen Benutzer welches Objekt im Innenraum bewegt wurde, und die Benutzeridentifikationsdaten dieses Benutzers gemeinsam mit den Objektsidentifikationsdaten dieses Objektes in dem Datenspeicher zu speichern.

Vorzugsweise ist die Datenverarbeitungseinrichtung dazu programmiert, aus dem Startbild, den Videodaten und/oder dem Endbild den Bewegungspfad, des mindestens einen Objektes innerhalb der Inkubatorkammer zu ermitteln und in Form von Bewegungshistoriedaten in dem Datenspeicher zu speichern, insbesondere zeitabhängig zu speichern. Vorzugsweise ist die Datenverarbeitungseinrichtung dazu programmiert, aus dem Startbild, den Videodaten und/oder dem Endbild eine Bewegungshistorie des mindestens einen Objektes innerhalb der Inkubatorkammer zu ermitteln und in Form von Bewegungshistoriedaten in dem Datenspeicher zu speichern, insbesondere zeitabhängig zu speichern, vorzugsweise mit Informationen über Anzahl und/oder Zeitpunkte der mittels Türsensor ermittelten Änderungen des Status der Türöffnung (offen/geschlossen) der Inkubatortüre. Der Bewegungspfad enthält vorzugsweise gespeicherte Positionsdaten des Objektes, wobei diese Positionsdaten den Bewegungspfad des Objektes markieren, insbesondere zwischen einem Startbild und einem Endbild, insbesondere zwischen einer -insbesondere unbewegten oder auch bewegten- Startposition des Objektes und einer -insbesondere unbewegten- Endposition. Die Bewegungshistoriedaten enthalten vorzugsweise zeitabhängig gespeicherte Positionsdaten bzw. Bewegungspfade, vorzugsweise innerhalb mindestens eines Zeitabschnitts oder während des gesamten Aufenthaltes dieses Objektes im Inkubator. Bewegungshistoriedaten können auch Informationen zu dem die Positionsänderung auslösenden Benutzer in Form von Benutzeridentifikationsdaten umfassen. Dies ist insbesondere bei Objekten, die wertvolle Proben enthalten, von Vorteil.

Vorzugsweise weist der Inkubator ein Display (=einen Bildschirm) auf. Der Bildschirm ist vorzugsweise ein fester Bestandteil des Inkubators, insbesondere der Inkubatortüre. Er kann aber auch entfernt vom Inkubator angeordnet sein und kann insbesondere Bestandteil eines Externgerätes sein, das in einer Datenaustauschverbindung mit der Datenverarbeitungseinrichtung des Inkubators stehen kann.

Vorzugsweise ist die Datenverarbeitungseinrichtung dazu programmiert, eine graphische Wiedergabe des Innenraums der Inkubatorkammer, insbesondere des mindestens einen Lagerbereichs, auf dem Bildschirm anzuzeigen. Die graphische Wiedergabe kann ein Foto des Lagerbereichs beinhalten, auf dem ein oder mehrere Bestandsobjekte des Inkubators angezeigt sein können. Der Lagerbereich kann insbesondere eine Lagerplatte im Inkubator sein oder ein vorbestimmter Ausschnitt desselben. Das Foto kann ein mit der Kamera aufgenommenes Bild zeigen, dass gegebenenfalls nachbearbeitet sein kann. Vorzugsweise beinhaltet diese Nachbearbeitung, dass ein von der Kamera verzerrt aufgenommenes Bild begradigt wird. Algorithmen für solch eine Nachbearbeitung sind allgemein bekannt und frei verfügbar (for example: OpenCV, "Omnidirectional Camera Calibration"). Die Verzerrung kann insbesondere optisch bedingt sein und auf die Verwendung einer Weitwinkel- bzw. Fischaugenoptik zurückzuführen sein.

Die graphische Wiedergabe kann insbesondere eine abstrahierte Darstellung eines von der Kamera aufgenommenen Bildes oder Bildausschnitts sein. Beispielsweise kann die graphische Wiedergabe eine aus der Vogelperspektive (oder einer anderen Perspektive) gezeigter abstrahierter Lagerbereich sein, insbesondere die graphische Darstellung eines Rechtecks oder die perspektivische Darstellung eines Quaders. Die Bestandsobjekte können ebenfalls in abstrahierter Form dargestellt sein, z.B. als rechteckförmige oder quaderförmige graphische Bildobjekte. Ziel einer solchen Darstellung ist es insbesondere, dem Benutzer über den Standort des Objektes/der Objekte im Inkubator bzw. im Lagerbereich zu informieren. Dies ermöglicht einen schnellen Zugriff des Benutzers auf das/die gewünschte(n) Objekt(e) und minimiert die Zeit, in der die Inkubatortüre geöffnet ist. Im Fall, dass die Differenzierung zwischen in einem Stapel aus Objekten enthaltenen Einzelobjekten ermöglicht werden soll, ist eine graphische Wiedergabe aus einer von der Vogelperspektive abweichenden Perspektive sinnvoll, beispielsweise aus einer seitlichen Perspektive, um einzelne Objekte eines Stapels graphisch hervorheben zu können.

Vorzugsweise ist die Datenverarbeitungseinrichtung dazu programmiert, graphisch anzuzeigen, wo das durch die Objektpositionsdaten identifizierte Objekt im Lagerbereich bzw. im Innenraum der Inkubatorkammer positioniert ist, bzw. um graphisch anzuzeigen, wo alle im Innenraum lokalisierten Objekte angeordnet sind.

Vorzugsweise ist die Datenverarbeitungseinrichtung dazu programmiert, in Abhängigkeit von mindestens einem Bedingungsparameter ein Objekt oder mehrere Objekte im Display graphisch hervorzuheben. Der Bedingungsparameter kann die Benutzeridentifikationsdaten bezeichnen. Das Hervorheben ist sowohl bei einer abstrahierten Darstellung als auch bei einer fotografischen Wiedergabe eines von der Kamera aufgenommenen Bildes oder Bildausschnitts des Lagerbereichs im Display des Inkubators möglich.

Vorzugsweise ist die Datenverarbeitungseinrichtung dazu programmiert, in Abhängigkeit von Benutzeridentifikationsdaten eines Benutzers (Einzelnutzer, einer Nutzergruppe, oder mehrere Benutzer) ein Objekt oder mehrere Objekte graphisch auf dem Display hervorzuheben, welches dem Benutzer als Eigentum zugeordnet ist, nämlich z.B. indem die benutzerbezogenen ID-Positionsdaten die Benutzeridentifikationsdaten dieses Benutzers enthalten. Eigentümer ist derjenige, der das Objekt betreut und -in den meisten Fällen selber oder mithilfe einer Hilfsperson- in die Inkubatorkammer eingestellt hat. Vorzugsweise ist die Datenverarbeitungseinrichtung dazu programmiert, ausgehend von vorgegebenen Benutzeridentifikationsdaten festzustellen, wo die diesen Benutzeridentifikationsdaten mittels der benutzerbezogenen Objektpositionsdaten zugeordneten Objekte positioniert sind und, insbesondere, diese Objekte graphisch hervorzuheben.

Der Bedingungsparameter kann auch Informationen über eine Zeitdauer oder einen Zeitpunkt beinhalten, z.B. die Zeitdauer mit der ein Objekt bereits in der Inkubatorkammer angeordnet war. Dadurch kann ein Benutzer sich schnell einen Überblick darüber verschaffen, wie lange ein oder mehrere Objekte bereits in der Inkubatorkammer lagern, eventuell dort von ihrem Eigentümer vergessen wurden. Oder der Inkubator kann abhängig von einem mittels eines Sensors des Inkubators detektierten Ereignisses oder abhängig von einem Zeitplan, der im Inkubator oder einem Externgerät gespeichert sein kann, ein oder mehrere Objekte graphisch hervorheben, die der Aufmerksamkeit des Benutzers bzw. des Laborpersonals bedürfen.

Oder der Bedingungsparameter kann, im Falle der Implementierung einer Objektklassenerkennung, Informationen über eine bestimmte Objektklasse beinhalten. Es können so ein oder mehrere Objekte derselben Objektklasse (oder der davon abweichenden Objektklassen) graphisch hervorgehoben werden, beispielsweise um den Standort aller Petrischalen (und nicht: Zellkulturflaschen) im Inkubatorinnenraum hervorzuheben.

Oder der Bedingungsparameter kann, im Falle der Implementierung einer Objektindividualerkennung, Informationen über ein bestimmtes Objektindividuum beinhalten. Auf diesem Weg lässt sich eine Objektsuche anhand von Individualmerkmalen umsetzen, indem z.B. der Inkubator Mittel zur Eingabe von Individualmerkmalen aufweist, z.B. einen Barcode, QR-Code, eine individuelle Beschriftung oder ein Foto des individuellen Objektes. Es kann so das individuelle Objekt graphisch hervorgehoben und leicht gefunden werden.

Vorzugsweise ist die Datenverarbeitungseinrichtung dazu programmiert, eine graphische Wiedergabe des Innenraums der Inkubatorkammer, insbesondere des mindestens einen Lagerbereichs, auf dem Bildschirm anzuzeigen und insbesondere, den im Inkubator verfügbaren freien Lagerplatz graphisch anzuzeigen bzw. hervorzuheben. Beispielsweise kann der Lagerbereich abstrahiert gezeigt werden und eine freie Lagerposition (oder mehrere verfügbare freie Lagerplätze) können graphisch hervorgehoben sein, indem der entsprechende Bereich z.B. in grüner oder weißer, oder einer zeitlich wechselnden (Blinken), Kontrastfarbe zum Hintergrund dargestellt wird. Auf diese Weise muss der Benutzer keine Zeit darauf verwenden, einen möglichen freien Lagerplatz zu suchen, oder einen solchen durch Bewegung von Bestandsobjekten zu schaffen.

Zudem kann, ähnlich der Funktion eines Parkwächters, die Datenverarbeitungseinrichtung dazu programmiert sein, die Belegung des Innenraums der Inkubatorkammer bzw. des mindestens einen Lagerbereichs zu planen und insbesondere auf diese Weise die Nutzung der verfügbaren Lagerfläche zu optimieren. Dazu kann die Datenverarbeitungseinrichtung dazu programmiert sein, vorbestimmte Abstände zwischen einem oder mehreren Bestandsobjekten zu einem neu einzustellenden Objekt zu berücksichtigen und insbesondere dem Benutzer vorzuschlagen, indem der freie Lagerplatz entsprechend als verfügbar und/oder nicht verfügbar hervorgehoben angezeigt wird. Gemäß dieser Beispiele kann der Inkubator ein computer-/softwareimplementiertes Planungsprogramm zur Belegung des Innenraums des Inkubators aufweisen, das insbesondere die Position mindestens eines Objekts im Innenraum (Bestandsobjekts) und/oder insbesondere den frei verfügbaren Lagerplatz berücksichtigt, eventuell auch die Zeitpunkte zu denen das mindestens eine Bestandsobjekt neu eingestellt wurde, oder Zeitpunkte in der Zukunft, an denen das Einstellen weiterer Objekte in den Inkubator geplant sind. Solche Zeitpunkte können insbesondere bekannt sein, wenn der Inkubator mit einem Laborinformationssystem (LIS) oder einem anderen (Labor-)Datenaustauschnetzwerk verbunden ist. Der Inkubator weist vorzugsweise einen Zeitgeber auf, eine Uhr, oder einen Zeitmesser.

Es ist möglich und besonders bevorzugt, dass eine Datenverarbeitungseinrichtung des Inkubators dazu programmiert ist, einen Belegungszustand des Innenraums der Inkubatorkammer zu ermitteln, und/oder vorzugsweise zu Durchführung einer oder mehrerer der folgenden Schritte programmiert ist, insbesondere in Abhängigkeit von den ID-Positionsdaten des mindestens einen im Innenraum angeordneten Objektes einen Belegungszustand des Innenraums der Inkubatorkammer zu ermitteln, in Abhängigkeit von den klassenbezogenen ID-Positionsdaten des mindestens einen im Innenraum angeordneten Objektes einen Belegungszustand des Innenraums der Inkubatorkammer zu ermitteln, in Abhängigkeit von den individualbezogenen ID-Positionsdaten des mindestens einen im Innenraum angeordneten Objektes einen Belegungszustand des Innenraums der Inkubatorkammer zu ermitteln.

Ein Belegungszustand des Innenraums kann durch Informationen definiert sein, die das im Innenraum von dem mindestens einen Objekt eingenommene Volumen beschreiben, und/oder die das im Innenraum von dem mindestens einen Objekt nicht eingenommene Volumen, also das freie Volumen beschreiben, und/oder die von dem mindestens einen Objekt eingenommene Lagerfläche auf mindestens einem Lagerbereich bzw. auf dem insgesamt verfügbaren Lagerbereich im Innenraum der Inkubatorkammer, und/oder die von dem mindestens einen Objekt nicht eingenommene, also die freie, Lagerfläche auf mindestens einem Lagerbereich bzw. auf dem insgesamt verfügbaren Lagerbereich im Innenraum der Inkubatorkammer, wobei diese Angaben jeweils auf das gesamte Innenraumvolumen bzw. die gesamte Lagerfläche bezogen sein können, wobei diese Informationen z.B. das Verhältnis eines nicht verfügbaren (belegten) oder eines freien (nicht belegten) Innenraumvolumens zum Gesamtvolumen des Innenraums beinhalten können, oder wobei diese Informationen z.B. das Verhältnis einer nicht verfügbaren (belegten) oder einer freien (nicht belegten) Lagerfläche zur Gesamtlagerfläche im Innenraum beinhalten können.

Belegungszustandsdaten, welche die Informationen über den Belegungszustand enthalten, können auch die ID-Positionsdaten, klassenbezogene ID-Positionsdaten und/oder individualbezogene ID-Positionsdaten enthalten. Auf diese Weise ist die Angabe einer Ortsauflösung der Belegung möglich, also die Angabe zur Lokalisation der Belegung im Innenraum, bzw. eine Dichteverteilung der Objekte im Innenraum.

Es ist möglich und besonders bevorzugt, dass eine Datenverarbeitungseinrichtung des Inkubators dazu programmiert ist, Informationen über den Belegungszustand des Inkubators in Form von Belegungszustandsdaten in einem Datenspeicher zu speichern, insbesondere auf ein externes Datenverarbeitungsgerät, insbesondere eines Laborgeräts, eines PC's,oder eines eines mobilen Computers, insbesondere ein Tabletcomputers oder eines Smartphones zu übertragen.

Es ist möglich und besonders bevorzugt, dass eine Datenverarbeitungseinrichtung des Inkubators dazu programmiert ist, Informationen über den Belegungszustand des Inkubators auf einem Bildschirm des Inkubators oder eines externen Datenverarbeitungsgeräts anzuzeigen, insbesondere in Abhängigkeit von Belegungszustandsdaten, die einem Datenspeicher entnommen sein können. Das externe Datenverarbeitungsgerät kann Teil eines Laborgeräts, PC's, eines mobilen Computers, insbesondere eines Tabletcomputers oder eines Smartphones sein.

In Testserien, die Ausführungsformen der vorliegenden Erfindung zugrunde liegen, wurde festgestellt, dass der sich nach einer Öffnung der Inkubatortüre aufgrund der Temperaturregelung ergebende zeitliche Temperaturverlauf in der Inkubatorkammer von dem Belegungszustand der Inkubatorkammer abhängt. Ist ein größeres Volumen des Kammerinnenraums durch Bestandsobjekte belegt, so liegt ein geringeres freies Kammerinnenraumvolumen vor, dass sich aus der Differenz des Kammerinnenraums und des von den Objekten eingenommenen Belegungsvolumens ergibt. Eine auf die Regelung des gesamten Innenraumvolumens ausgelegte Temperaturregelung wird in dieser Situation eventuell andere, nicht erwünschte Resultate erzielen. Es kann zu einem raschen Überschwingen kommen, das unerwünscht ist, auch wenn dabei die Wiederherstellung der Zieltemperatur, z.B. 37 °C, beschleunigt sein kann, d.h. auch wenn sich die Erholungszeit dabei verkürzt. Wenn mehrere neue Objekte mit niedrigeren Temperaturen als der Zieltemperatur neu eingestellt werden, kann es auch zu einer Verzögerung der Erholungszeit kommen, die Kenntnis über kältere, neu eingestellte Objekte kann aber ebenfalls zu einer Anpassung der Temperaturregelung verwendet werden. Die Temperaturregelung der Temperatur im Innenraum der Inkubatorkammer hängt von Regelungsparametern ab.

Vorzugsweise ist eine elektronische Steuereinrichtung des Inkubators dazu eingerichtet bzw. programmiert, dass mindestens eine Temperiereinrichtung des Inkubators, die zur Temperierung der Inkubatorkammer angeordnet ist, während der Temperaturregelung in Abhängigkeit von der Zeit t mit der elektrischen Leistung Ptemp(t) betrieben wird. Insbesondere kann der Inkubator dazu eingerichtet sein, dass die Temperiereinrichtung mittels einer Pulsweitenmodulation (PWM) des Stroms betrieben wird. Die Leistung wird dann insbesondere durch den Tastgrad der PWM bestimmt, da die Amplitude des Stroms vorzugsweise konstant ist. Insbesondere die genannten Größen können Variablen der Temperaturregelung sein, also Regelungsparameter.

Vorzugsweise ist eine elektronische Steuereinrichtung des Inkubators dazu eingerichtet bzw. programmiert, dass die Temperaturregelung oder die Regelung der Inkubatorgaszuführung (z.B. CO₂, N₂, und/oder O₂), insbesondere mindestens ein Regelungsparameter, in Abhängigkeit vom Belegungszustand der Inkubators angepasst werden kann. Auf diese Weise lässt sich der Einfluss von im Innenraum der Inkubatorkammer angeordneten Objekten auf das Ansprechverhaltendes geregelten Systems berücksichtigen. Insbesondere lässt sich die Erholungszeit im Falle einer größeren Belegung des Innenraums reduzieren.

Die Datenverarbeitungseinrichtung des Bilderfassungssystems oder anderen Systems ist vorzugsweise separat zu einer ersten Datenverarbeitungseinrichtung des Inkubators. Sie kann aber auch Bestandteil der Steuereinrichtung des Inkubators (auch bezeichnet als "erste Steuereinrichtung") sein, die Funktionen des Inkubators steuert. Die Funktionen der Steuereinrichtung sind insbesondere durch elektronische Schaltkreise implementiert. Die Datenverarbeitungseinrichtung des Bilderfassungssystems kann mindestens eine CPU, und optional mindestens eine GPU aufweisen. Eine GPU kann zur Bildverarbeitung oder der Durchführung von Deep Learning-Prozessen vorgesehen sein. Alternativ zu einer CPU, oder einer GPU kann die Datenverarbeitungseinrichtung auch einen dedizierten Chip, z.B. den Nvidia Jetson, zur Bildverarbeitung oder der Durchführung von DeepLearning-Prozessen aufweisen, die vorzugsweise bei der Objektverfolgung, insbesondere bei einer möglichen Objektklassifizierung oder Objektindividualerkennung verwendet werden können. Solche dedizierten Chips kann man als Rechenbeschleuniger zur Datenverarbeitungseinrichtung hinzufügen. Eine GPU ist auf vielen System on a Chip Systemen (SoC) schon vorhanden (für die Widergabe von Grafik und Videos). Ein Raspberry PI kann ebenfalls als Teil des SOCs eine dedizierte GPU Einheit aufweisen.

Das Objektverfolgungssystem kann eine Steuerungseinrichtung aufweisen, die separat zur ersten Steuereinrichtung vorgesehen sein kann. Die Begriffe "Steuereinrichtung" und "Steuerungseinrichtung" werden in dieser Beschreibung synonym verwendet. Eine Steuereinrichtung kann einen Mikroprozessor aufweisen, der die Datenverarbeitungseinrichtung beinhalten kann. Der Mikroprozessor kann vom Typ "Rasberry Pi" sein. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird -jeweils bezogen auf den Inkubator und/oder das Objektverfolgungssystem. Die Funktionen des Inkubators und/oder des Objektverfolgungssystems und/oder der Steuereinrichtung und/oder der Datenverarbeitungseinrichtung können in Verfahrensschritten beschrieben werden. Sie können als Bestandteile des Steuerungsprogramms realisiert sein, insbesondere als Unterprogramme des Steuerungsprogramms.

Eine Steuereinrichtung weist im Rahmen der vorliegenden Erfindung generell insbesondere die Datenverarbeitungseinrichtung, insbesondere eine Recheneinheit (CPU) zum Verarbeiten von Daten und/oder einen Mikroprozessor auf oder ist die Datenverarbeitungseinrichtung. Die Datenverarbeitungseinrichtung der Steuereinrichtung des Inkubators kann vorzugsweise auch zum Steuern des Objektverfolgungssystems eingerichtet sein.

Die Datenverarbeitungseinrichtung des Bilderfassungssystems ist vorzugsweise eine außerhalb der Inkubatorkammer oder des Inkubators und insbesondere optional getrennt von dieser/diesem angeordnete Vorrichtung, auch als externe Vorrichtung bzw. externe Datenverarbeitungseinrichtung bezeichnet. Die Datenverarbeitungseinrichtung und der Inkubator stehen vorzugsweise in einer Datenverbindung und sind vorzugsweise Bestandteile eines Netzwerks zum Datenaustausch.

Die mindestens eine Kamera des Bilderfassungssystems ist vorzugsweise über eine Kabelverbindung mit der Steuereinrichtung bzw. Datenverarbeitungseinrichtung des Bilderfassungssystems verbunden. Dazu weist die Inkubatorkammer eine Durchgangsöffnung (Port) auf, durch die das Kabel der Kabelverbindung geführt ist. Dabei ist vorzugsweise eine Dichtung, insbesondere Silikondichtung vorgesehen, die den Port abdichtet, um eine Beeinflussung der Atmosphäre im Inkubator (weitestgehend) zu verhindern. Alternativ ist die Kamera für einen kabellosen Datenaustausch mit der Steuereinrichtung bzw. Datenverarbeitungseinrichtung verbunden, z.B. per Bluetooth oder WLAN.

Der Inkubator kann ein Teilgehäuse aufweisen, in dem insbesondere mindestens eine Steuerungseinrichtung (des Inkubators und/oder des Objektverfolgungssystems) angeordnet ist. Das Teilgehäuse ist vorzugsweise an der Rückseite des Inkubators, also insbesondere gegenüberliegend der Inkubatortüre angeordnet.

Das System, der Inkubator und/oder das Bilderfassungssystem und/oder die Datenverarbeitungseinrichtung und/oder die Steuerungseinrichtung sind vorzugsweise dazu eingerichtet, die Positionsdaten des mindestens einen Objektes oder einer Vielzahl von Objekten zur Bildung einer elektronischen Dokumentationsdatei zu verwenden, in der die Positionen und/oder Bewegung der Objekte und/oder deren Aufenthaltszeit und/oder die Identifikationsdaten des die Bewegung auslösenden Benutzers im Inkubator protokolliert und dokumentiert werden. Diese Dokumentationsdatei wird dann insbesondere in einer Datenspeichereinrichtung gespeichert und vorzugsweise laufend aktualisiert. Auf diese Weise kann ein "korrekter" Umgang mit den Objekten gemäß Standardprotokollen bei Bedarf zertifiziert werden. Andererseits können nachträglich Abweichungen von Standardprotokollen ermittelt werden und/oder Informationskorrelationen ermittelt werden. Durch die Erfassung solcher Daten kann die Qualität von zellbasierten Laborarbeiten bzw. medizinischen, biologischen und pharmazeutischen Verfahren erheblich verbessert werden und zuverlässiger werden. Die Reproduzierbarkeit der zell-basierten Laborarbeiten kann erhöht werden, Abweichungen von normalen Eigenschaften können frühzeitig erkannt werden, um dem Anwender die Möglichkeit einer Korrektur oder frühzeitigen Wiederholung des Experiments zu geben. Die Dokumentationsdatei kann dem Benutzer oder einer externen Datenverarbeitungseinrichtung von der Steuerungseinrichtung über Datenaustausch bereitgestellt werden. Insbesondere in kritischen Anwendungen, die z.B. einen forensischen Bezug haben oder bei denen Zellen erheblichen Wertes kultiviert werden, ist eine solche Dokumentation besonders sinnvoll.

Die Erfindung betrifft insbesondere auch ein System zur Inkubation lebender Zellkulturen, aufweisend einen Inkubator zur Inkubation lebender Zellkulturen, der aufweist:
eine Inkubatorkammer zur Aufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt, eine Inkubatortüre zum Verschließen der Kammeröffnung,
ein zum Nachrüsten des Inkubators eingerichtetes bildverarbeitendes Bilderfassungssystem, das eine Datenverarbeitungseinrichtung mit einem Datenspeicher, eine Beleuchtungseinrichtung, und eine Kameraeinrichtung aufweist, die dazu eingerichtet ist, mindestens einen Lagerbereich der Inkubatorkammer zu erfassen,
wobei das Bilderfassungssystem eingerichtet ist, und insbesondere die Datenverarbeitungseinrichtung dazu programmiert ist,
   - mittels der Beleuchtungseinrichtung den sich zwischen den Innenwänden erstreckenden Lagerbereich des Inkubators zu beleuchten,
   - mittels der Kameraeinrichtung mindestens ein Bild des sich zwischen den Innenwänden erstreckenden Lagerbereich zu erfassen, und
   - das mindestens eine Bild mittels der Datenverarbeitungseinrichtung in Form von Bilddaten in der Datenspeichereinrichtung zu speichern.

Das vorstehend genannte System beruht somit auf einem Inkubator, der mit einem nachrüstbaren Bilderfassungssystem nachrüstbar ist, so wie dieses im Anspruch 1 fester Bestandteil des Inkubators ist, wobei das nachrüstbare Bilderfassungssystem entsprechend kompatibel sein muss mit dem so bezeichneten "kompatiblen Inkubator". "Nachrüsten" beinhaltet vorzugsweise jeweils, dass die Kameraeinrichtung in der Inkubatorkammer geeignet anordenbar bzw. befestigbar ist, dass die Beleuchtungseinrichtung in der Inkubatorkammer geeignet anordenbar bzw. befestigbar ist, dass insbesondere die Datenverarbeitungseinrichtung mit Datenspeicher im/am Inkubator geeignet anordenbar bzw. befestigbar ist, dass insbesondere die Kameraeinrichtung und/oder die Beleuchtungseinrichtung und/oder die Datenverarbeitungseinrichtung mit Datenspeicher zum Zweck der Datenübertragung an eine Datenverarbeitungseinrichtung eines Inkubators oder eines externen Computers angeschlossen sind bzw. anschließbar sind, dass insbesondere die Kameraeinrichtung und/oder die Beleuchtungseinrichtung und/oder die Datenverarbeitungseinrichtung mit Datenspeicher an eine Energieversorgung angeschlossen sind bzw. anschließbar sind, die Teil des Bilderfassungssystems oder des Inkubators sein kann. Es kann auch vorgesehen sein, insbesondere alternativ oder zusätzlich, dass die Datenverarbeitungseinrichtung des Bilderfassungssystems durch eine Datenverarbeitungseinrichtung des Inkubators gebildet wird, indem die Kameraeinrichtung und/oder die Beleuchtungseinrichtung zum Zweck der Datenübertragung an eine Datenverarbeitungseinrichtung des Inkubators angeschlossen sind bzw. anschließbar sind.

Vorzugsweise ist eine Steuereinrichtung des erfindungsgemäßen Inkubators oder des kompatiblen Inkubators, die insbesondere auch die atmosphärischen Parameter in der Inkubatorkammer steuern kann (Temperatur, Gaspartialdruck CO₂, H₂O etc.) bzw. deren Datenverarbeitungseinrichtung dazu eingerichtet bzw. programmiert, in Abhängigkeit von Daten aus dem Bilderfassungssystem, insbesondere von Positionsdaten bzw. von der Endposition mindestens einen Objektes im Lagerbereich, mindestens einen Betriebsparameter des Inkubators zu bestimmen, insbesondere einen Parameter, der die Anzeige von Informationen auf einem Bildschirm des Inkubators steuert oder einen Parameter, der auf dem Bildschirm des Inkubators angezeigt wird. Insbesondere können Positionsdaten bzw. die Endposition mindestens eines Objektes auf dem Bildschirm wiedergegeben werden. Vorzugsweise weist das System zur Inkubation lebender Zellkulturen auf: ein getrennt vom Inkubator mit diesem in Datenaustauschverbindung stehendes Externgerät, insbesondere eine -insbesondere mobile- Benutzeridentifikationseinrichtung und insbesondere eine Datenaustauscheinrichtung, mittels der die Datenverarbeitungseinrichtung mit dem Externgerät Daten austauschen kann, insbesondere anhand der Benutzeridentifikationseinrichtung Benutzeridentifikationsdaten ermitteln kann.

Die Erfindung bezieht sich auch auf ein Verfahren zur Bilderfassung in einem Inkubator, welcher der Inkubation lebender Zellkulturen dient, und der aufweist:
- eine Inkubatorkammer zur Aufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt,
- eine Inkubatortüre zum Verschließen der Kammeröffnung,
- ein Bilderfassungssystem, aufweisend
   ∘ eine Beleuchtungseinrichtung,
   ∘ eine Kameraeinrichtung und
   ∘ eine Datenverarbeitungseinrichtung mit einem Datenspeicher,
   wobei das Verfahren die Schritte aufweist:
   - Beleuchten des sich zwischen den Innenwänden erstreckenden Lagerbereichs mittels der Beleuchtungseinrichtung,
   - Erfassen mindestens eines Bildes des sich zwischen den Innenwänden erstreckenden Lagerbereichs während der Beleuchtung mittels der Kameraeinrichtung, und
   - Speichern des mindestens einen Bildes in Form von Bilddaten in der Datenspeichereinrichtung.

Die Erfindung bezieht sich auch auf ein, insbesondere zum Nachrüsten eines Inkubators eingerichtetes, bildverarbeitendes Bilderfassungssystem, aufweisend
- eine Beleuchtungseinrichtung,
- mindestens eine Kameraeinrichtung und
- eine Datenverarbeitungseinrichtung mit einem Datenspeicher,
   wobei das Bilderfassungssystem dazu eingerichtet ist,
- mittels der Beleuchtungseinrichtung den sich zwischen den Innenwänden erstreckenden Lagerbereich zu beleuchten,
- mittels der Kameraeinrichtung mindestens ein Bild des sich zwischen den Innenwänden erstreckenden Lagerbereich zu erfassen, und
- das mindestens eine Bild mittels der Datenverarbeitungseinrichtung in Form von Bilddaten in der Datenspeichereinrichtung zu speichern.

Weitere bevorzugte Ausgestaltungen des erfindungsgemäßen Verfahrens lassen sich aus der Beschreibung des erfindungsgemäßen Inkubators und dessen bevorzugten Ausgestaltungen entnehmen. Ferner ergeben sich weitere Ausgestaltungsoptionen der Erfindung aus den Ausführungsbeispielen in den Figuren. Gleiche Teile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1 zeigt einen erfindungsgemäßen Inkubator gemäß einem Ausführungsbeispiel in perspektivischer Ansicht.
Fig. 2 zeigt den Inkubator aus Fig. 1 in einer Frontansicht.
Fig. 3 zeigt den Inkubator aus Fig. 1 in einer Frontansicht mit graphischer Darstellung der Belegung der Inkubatorkammer mit Objekten, die benutzerbezogen farbcodiert hervorgehoben sind.
Fig. 4a zeigt ein Smartphone mit Kamera und Display 63 als Externgerät, das Bestandteil eines Systems 400 umfassend den Inkubator 1 aus Fig. 3 und das Smartphone 69 sein kann.
Fig. 4b zeigt eine Legende der im Bildschirm der Fig. 3 verwendeten Farbcodierung zur Hervorhebung benutzerbezogener Objekte.
Fig. 5a zeigt in einer schematischen Seitenansicht ein Bilderfassungssystem als Bestandteil des Inkubators aus den Fig. 1 bis 4b, in einem Beispiel einer Kammer mit einer einzigen überwachten Lagerplatte.
Fig. 5b zeigt in einer schematischen Seitenansicht ein Bilderfassungssystem als Bestandteil des Inkubators aus den Fig. 1 bis 4b, in einem Beispiel einer Kammer mit mehreren überwachten Lagerplatten.
Fig. 5c zeigt eine perspektivische Ansicht eines Lagerbereichs, der vom Objektverfolgungssystem der Fig. 5a und 5b überwacht wird, sowie die auf ein Koordinatensystem bezogene Startposition P1, Positionsänderungen dP und Endposition P2 eines verfolgten Objektes.
Fig. 5d zeigt ein von der Weitwinkel-Fischaugenkamera des in Fig. 5a und 5b verwendeten Bilderfassungssystems erfasstes, aufgrund der Optik verzerrt erscheinendes digitales Bild.
Fig. 5e zeigt das Bild der Fig. 5d, das vom Bilderfassungssystem durch Begradigungsalgorithmen entzerrt wurde.
Fig. 5f zeigt ein von der Weitwinkel-Fischaugenkamera des in Fig. 5a und 5b verwendeten Bilderfassungssystems erfasstes Standbild zur Ausgabe auf einem Bildschirm des Inkubators, bei dem die Bounding Boxes des Bilderfassungssystems, Identifikationsnummern und eine den Benutzer/Eigentümer identifizierende Farbkodierung gezeigt sind.
Fig. 5g zeigt einen möglichen Bildschirminhalt, der zur Erläuterung der in Fig. 5f gezeigten Bildschirmseite auf einem Bildschirm des Inkubators angezeigt werden kann.
Fig. 6 zeigt in einer schematischen Aufsicht einen Lagerbereich des Inkubators aus Fig. 1 bis 5f, inklusive Objekten, der in einem Bilderfassungsausschnitt einer Kamera des Bilderfassungssystems angeordnet ist.
Fig. 7 zeigt anhand des Ausschnitts aus Fig. 6 das Erfassen eines zwischen zwei Bestandsobjekte neu in den Inkubator eingestelltes Objekt.
Fig. 8 zeigt schematisch den Ablauf eines beispielhaften erfindungsgemäßen Verfahrens.

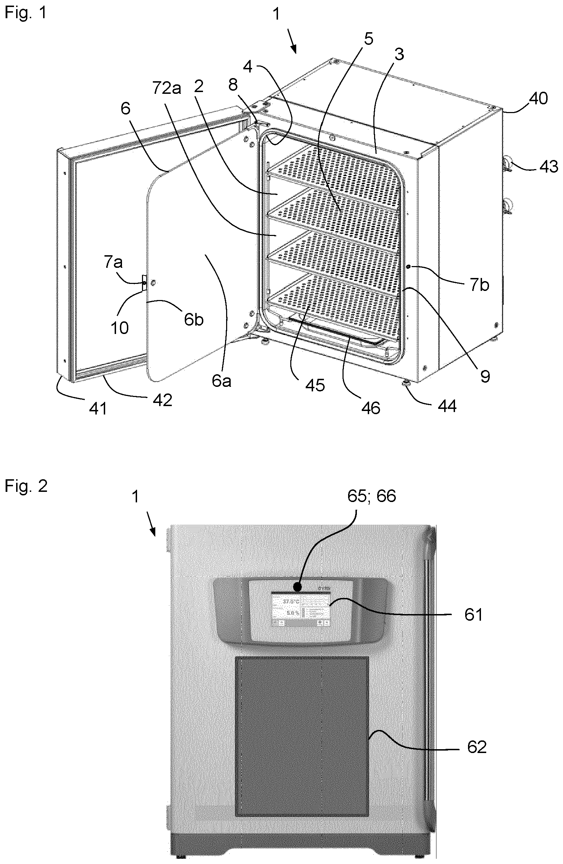

Fig. 1 zeigt einen Inkubator 1 zum Lagern von Laborproben, genauer gesagt einen CO2-Inkubator zur Lagerung von lebenden Zellkulturen in einer definierten Atmosphäre bei einer geregelten Temperatur, z.B. 37° C. Zu diesem Zweck ist der Kammerinnenraum 5 des Inkubators thermisch isoliert und gasdicht gegenüber der Umgebung abschließbar, die Gaszusammensetzung im Innenraum ist ebenfalls geregelt und kann über Gasanschlüsse 43 geändert werden. Das Kammergehäuse 2 des Inkubators steht auf Sockeln 44, kapselt den Innenraum 5 ein und mündet in der Frontseite 3 des Inkubators. Die Frontseite weist die Kammeröffnung 4 auf, durch die der Kammerinnenraum 5 zugänglich ist. Eine durchsichtige innere Kammertüre 6 dient dem Schließen der Kammeröffnung in einer Schließposition der Kammertüre. Beim Inkubator 1 ist das Kammergehäuse 2 innerhalb des Innenraums eines Außengehäuses 40 platziert, so dass das Kammergehäuse 2 und das Außengehäuse 40 voneinander beabstandet und gegeneinander thermisch isoliert sind. Im Kammerinnenraum sind Regalblecheinsätze 45 und eine Luftbefeuchterwanne 46 zu sehen. Die Frontseite 3 des Kammergehäuses und die Frontseite des Außengehäuses fallen vorliegend zusammen.

Die äußere Inkubatortüre 41 und die Kammertüre 6 sind in einer geöffneten Position gezeigt. Die Außentüre 41 ist über Scharniere an der Außenkante des Außengehäuses schwenkbar gelagert und weist eine umlaufende Dichtung, insbesondere Silikondichtung 42, auf.

Wenn die Außentüre 41 geöffnet wurde, ist die innere Kammertüre 6 des Inkubators zunächst noch geschlossen. Dazu dient die Verschlusseinrichtung (10, 7a, 7b). Bei geschlossener Kammertüre 6 kann der Benutzer zunächst den Innenraum 5 durch die transparente Türwand sichten, bevor er die Tür öffnet und eine Laborprobe einsetzt oder entnimmt. Dennoch stellt das Öffnen der äußeren Inkubatortüre 41 bereits eine Störung dar, die die Inkubatoratmosphäre potentiell schädigen kann.

Der Inkubator weist eine in die Türe 41 verbaute und nach vorne ausgerichtete Außenkamera 65 auf, deren Bilder von der geeignet programmierten Datenverarbeitungseinrichtung des Inkubators auswertbar sind, insbesondere um einen Benutzer mittels Gesichtserkennung zu identifizieren, wodurch die mit der Datenverarbeitungseinrichtung verbundene Außenkamera 65 als Benutzeridentifikationseinrichtung 66 dient. Letzteres kann auch über die Kamera des Smartphones 69 erfolgen.

Um die gelagerten Laborproben zu schützen, ist es beim Inkubator wirksam, die Zeit zu minimieren, während der der Innenraum des Inkubators gegenüber der Umgebung exponiert ist (Öffnungszeitintervalle). Der vorliegenden Erfindung liegt die Beobachtung zugrunde, dass die Öffnungszeitintervalle durch ein Bilderfassungssystem 200 reduziert werden können. Der Inkubator 1 weist ein Bilderfassungssystem auf (nicht gezeigt in Fig. 1, 2).

Die Außenseite der äußeren Inkubatortüre weist, wie in Fig. 2 gezeigt ist, einen ersten Bildschirm auf, einen Touchscreen 61, über den Betriebsparameter des Inkubators 1 angezeigt werden, z.B. die Temperatur der Inkubatoratmosphäre oder einen Gaspartialdruck im Innenraum 5.

Die Außenseite der äußeren Inkubatortüre 41 weist einen zweiten Bildschirm 62 auf, der ein Touchscreen sein kann. Anstelle eines zweiten Bildschirms können aber auch alle Bildschirmausgaben auf einem einzigen Bildschirm erfolgen. Die Datenverarbeitungseinrichtung (nicht gezeigt) des Inkubators 1 ist dazu programmiert, im Bildschirm 62 die Belegung des Innenraums des Inkubators anzuzeigen. Der Bildschirm 62 dient als "digitales Fenster", das dem Benutzer den (virtuellen) Blick ins Inkubatorinnere ermöglicht. Dabei kann die graphische Wiedergabe des Innenraums bzw. des mindestens einen Lagerbereichs des Inkubators und dessen Belegung mit Bestandsobjekten so programmiert sein, dass bestimmte Bestandsobjekte abhängig von bestimmten Kriterien bzw. Bedingungsparametern graphisch hervorgehoben sind.

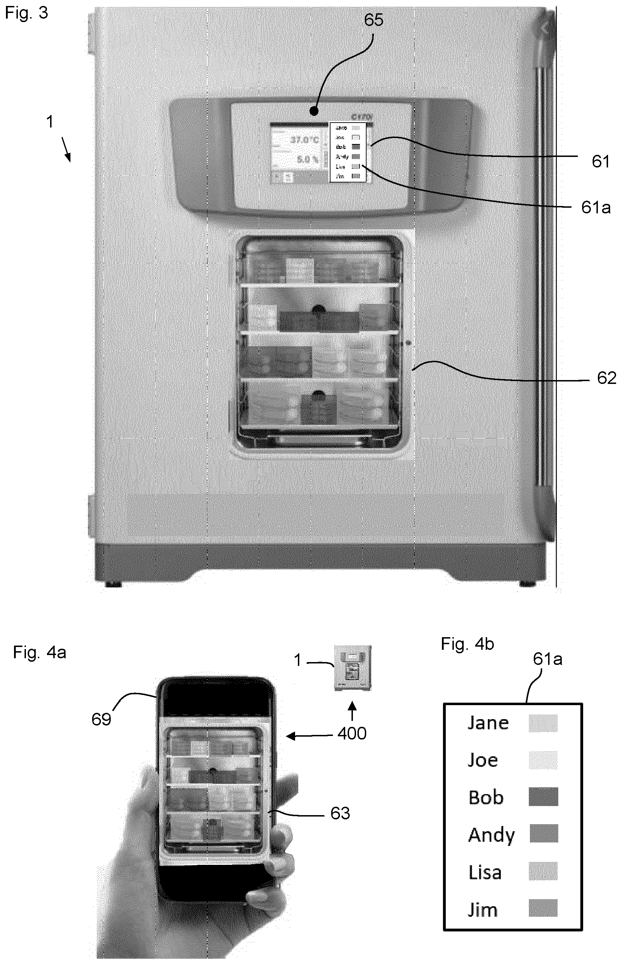

Zu Fig. 3: Die Datenverarbeitungseinrichtung des Inkubators 1 ist dazu programmiert, in Abhängigkeit von mindestens einem Bedingungsparameter, der hier von Benutzeridentifikationsdaten abhängt, ein Objekt oder mehrere Objekte im Display 62 anzuzeigen, und zwar gemäß deren jeweiliger Position im Innenraum des Inkubators, die mittels des Bilderfassungssystem ermittelt wurde. Es sind jeweils die mit bestimmten Benutzeridentifikationsdaten, die einen bestimmten Benutzer kennzeichnen, assoziierten Bestandsobjekte mit einer bestimmten nutzerabhängigen Farbe farblich hervorgehoben. Die Legende 61a zu dieser Art von Farbcodierung ist dem Benutzer hier über das obere Display 61 in dessen Unter-Bereich 61a angezeigt. Die Legende 61a ist in Fig. 4b größer gezeigt: den Benutzerkennungen "Jane", Joe" etc. sind die entsprechenden, im Display 62, 63 verwendeten Hervorhebungsfarben zugeordnet.

In Fig. 4 ist gezeigt, dass das Ausgabedisplay 61 und/oder 62 auch -alternativ oder zusätzlich- Bestandteil(e) eines Externgeräts sein kann/können, hier ein Smartphone 69, das in einer Datenaustauschverbindung mit dem Inkubator steht und das Display 63 aufweist, das hier als Bestandteil eines Inkubatorsystems fungiert.

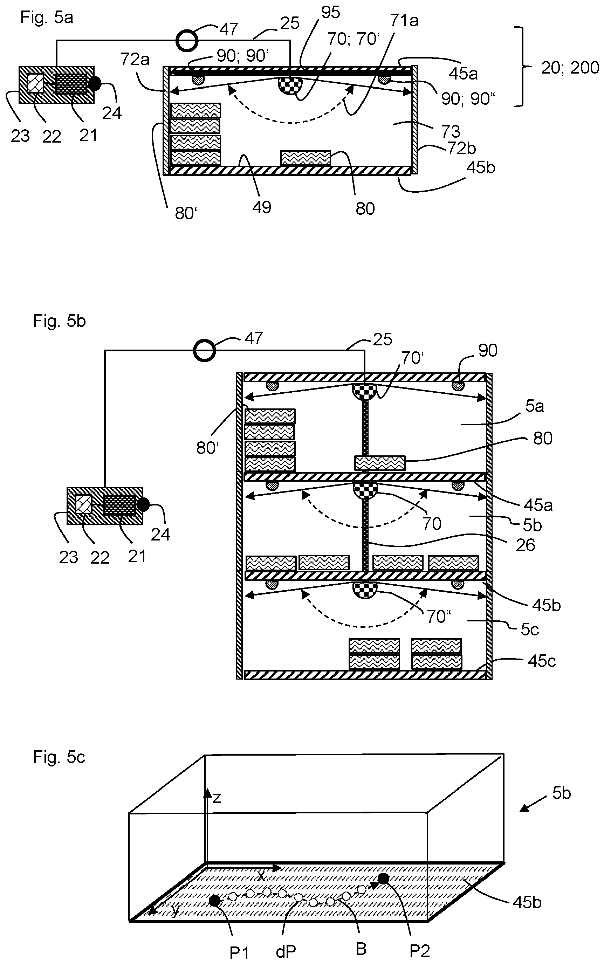

Fig. 5a zeigt in einer schematischen Frontansicht als Lagerplatten für Objekte die Regalblecheinsätze 45a und 45b des Inkubators 1, die übereinander angeordnet sind. Der vertikale Abstand solcher Regalblecheinsätze 45 in Inkubatoren ist meist nicht groß und liegt z.B. zwischen 10 und 40 cm, beim Inkubator 1 etwa bei 15 cm. Dadurch müssen entweder mehrere Kameras verwendet werden, um den gesamten Lagerbereich 45, hier die gesamte Lagerfläche des Regaleinsatzes 45b und den darüber liegenden "Luftraum" bis zum Regaleinsatz 45a zu erfassen. Die Kamera 70 bzw. Kameraeinrichtung 70' ist oder beinhaltet eine Weitwinkel- bzw. weitwinklige Fischaugenoptik-Kamera mit einem diagonal gemessenen Bildwinkel von ca. 200°.

Fig. 5a zeigt das im Inkubator 1 eingebaute Bilderfassungssystem 20, das im Falle der Ausführung als Nachrüstsystem auch mit dem Bezugszeichen 200 bezeichnet ist. Das Bilderfassungssystem 20 beinhaltet die Kamera 70, eine Weitwinkel-Fischaugenkamera, die im Sichtbereich bzw. Blickwinkel 71a von vorzugsweise 160° bis 220° den unter ihr liegenden Lagerbereich des Regalblecheinsatzes 45b und einen großen Teil von ca. 80% der Flächen der Inkubator-Innenwandabschnitte 72a, 72b erfasst, die mit den sich zwischen den Innenwänden 72a und 72b erstreckenden Lagerplatten 45a und 45b das Kompartiment 73 der Inkubatorkammer umgrenzen. Der weite Blickwinkel ermöglicht es, mit einer einzigen Kamera auszukommen, um den gesamten Lagerbereich des unten liegenden Regalblecheinsatzes 45b zu erfassen, insbesondere auch den Luftraum, in den die (Bestands-)Objekte 80' und 80 hineinragen, nämlich ein Stapel 80' von Zellkulturbehältern, und ein Zellkulturbehälter 80. Der nominale Sichtwinkel der Weitwinkel-Fischaugenkamera beträgt hier 200°, es wird aber nur ein Bildbereich ausgewertet, der einem Sichtwinkel entnommen aus dem Bereich von vorzugsweise 160° bis 170° entspricht.

Die Kamera ist vertikal über dem geometrischen Zentrum der Lagerfläche des Regalblecheinsatzes 45b angeordnet. Das Bilderfassungssystem 20 beinhaltet auch die Beleuchtungseinrichtung 90, die Steuereinrichtung 23, welche eine Datenverarbeitungseinrichtung 21 und einen Datenspeicher 22 als weitere Bestandteile des Bilderfassungssystems 20 aufweist. Die Datenverarbeitungseinrichtung 21 bzw. die Steuereinrichtung 23 sind über eine Kabelverbindung 25, die durch den Port 47 in der Inkubatorkammerrückwand in die Inkubatorkammer gelangt, mit der Kamera 70 und den in Fig. 5a nicht gezeigten weiteren Kameras verbunden, die jeweils vorgesehen sind, damit alle Lagerbereiche (alle Oberseiten von Regalblecheinsätzen 45, siehe Fig. 1) überwacht werden. Die Steuereinrichtung 23 weist zudem eine Datenschnittstelle 24 auf, über die eine Datenverbindung zu weiteren Inkubatorgerätebestandteilen ermöglicht wird, z.B. um Daten bzw. Signale an ein Display 61, 62, 63 des Inkubators auszugeben. Eine Beleuchtungseinrichtung 90 mit mehreren LEDs 90', 90" ist oberhalb der Lagerplatte 45b montiert und über Leitungen 25 mit der Steuereinrichtung 23 verbunden. Anstelle der gezeigten zwei LEDs kann eine Vielzahl von LEDs vorgesehen sein. Mittels der optionalen Beleuchtungseinrichtung 90 kann zum Zwecke der Bilderfassung der Lagerbereich 45b beleuchtet werden, falls dies zweckmäßig ist.

Fig. 5b zeigt in einer schematischen Seitenansicht ein Bilderfassungssystem als Bestandteil des Inkubators aus den Fig. 1 bis 4b in einem Beispiel einer Kammer mit mehreren überwachten Lagerplatten 45a, 45b, 45c. Die Darstellung ist eine Erweiterung des Prinzips aus Fig. 5a, bei dem die Inkubatorkammer in mehrere Kompartimente 5a, 5b und 5c unterteilt ist, die hier übereinander angeordnet sind und zum Gasaustausch verbunden sind, was über Löcher in den Lagerplatten 45a, 45b, 45c gebildet ist. Der Lagerbereich bzw. die Lagerplatte 45a im Kompartiment 5a wird von der Kamera 70' überwacht, der Lagerbereich bzw. die Lagerplatte 45b im Kompartiment 5b wird von der Kamera 70 überwacht, und der Lagerbereich bzw. die Lagerplatte 45c im Kompartiment 5c wird von der Kamera 70" überwacht, wobei die Kameras 70' und 70" analog zur Kamera 70 in Fig. 5a ausgeführt und angeordnet sind. Alle Kameras sind über ein Verbindungskabelbündel 26 im Inneren der Inkubatorkammer, das in die bereits in Figur 5a gezeigte Kabelverbindung 25 übergeht und durch den Port 47 in der Inkubatorkammerrückwand die Inkubatorkammer verlässt, mit der Steuereinrichtung 23 verbunden, deren Datenverarbeitungseinrichtung 21 zur Überwachung aller Objekte in allen drei Kompartimenten 5a, 5b und 5c eingerichtet ist. Das Bilderfassungssystem 20 des zu Fig. 5b gehörenden Inkubators umfasst hier drei Kameras 70, 70', 70", die Beleuchtungseinrichtung 90, die Datenverarbeitungseinrichtung 21, die Datenspeichereinrichtung 22 und die Verbindungsleitungen.

Fig. 5c zeigt eine perspektivische Ansicht eines Kompartiments 5b bzw. Lagerbereichs 45b, der vom Bilderfassungssystem der Fig. 5a und 5b überwacht wird, sowie die auf ein kartesisches Koordinatensystem (x, y, z) bezogenen Objektpositionen P1, P2. Im Falle, dass das Bilderfassungssystem durch Erfassen von Videodaten und mit Mitteln der digitalen Bildverarbeitung als Objektverfolgungssystem ausgeführt ist, können auch Positionsänderungen dP eines auf seinem Bewegungspfad B bewegten Objektes verfolgt werden. Der Ursprung des Koordinatensystems kann in einer Ecke des Kompartiments fest lokalisiert sein.

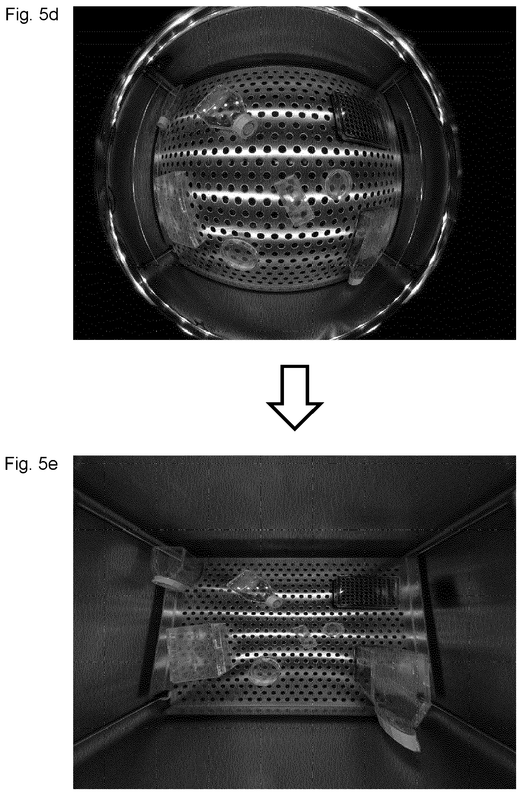

Fig. 5d zeigt ein von der Weitwinkel-Fischaugenkamera des in Fig. 5a und 5b verwendeten Bilderfassungssystems erfasstes, aufgrund der Optik verzerrt erscheinendes digitales Bild.

Fig. 5e zeigt das Bild der Fig. 5d, das vom Bilderfassungssystem durch Begradigungsalgorithmen entzerrt wurde.

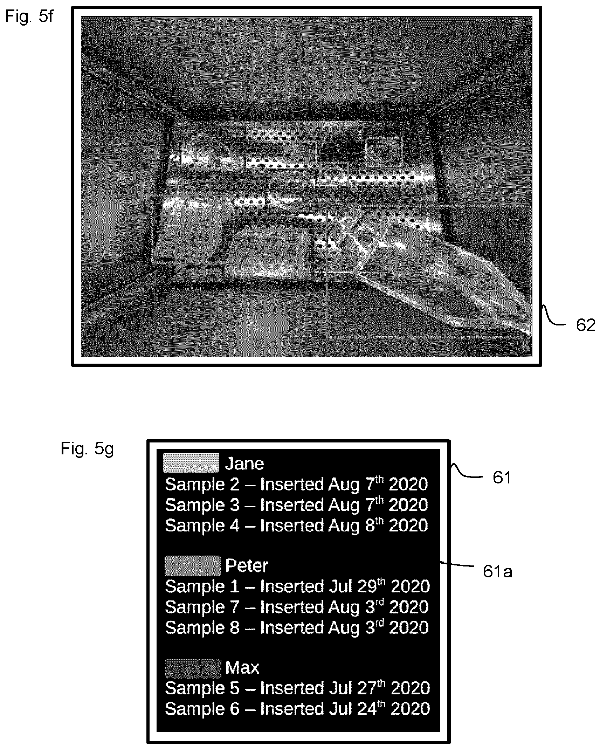

Fig. 5f zeigt ein von der Weitwinkel-Fischaugenkamera des in Fig. 5a und 5b verwendeten Bilderfassungssystems erfasstes Standbild zur Ausgabe auf einem Bildschirm des Inkubators, bei dem die Bounding Boxes des Bilderfassungssystems, Identifikationsnummern und eine den Benutzer/Eigentümer identifizierende Farbkodierung gezeigt sind.

Fig. 5g zeigt einen möglichen Bildschirminhalt, der zur Erläuterung der in Fig. 5f gezeigten Bildschirmseite auf einem Bildschirm des Inkubators angezeigt werden kann. Neben der Identifizierung der Objekte durch Identifikationsnummern ist auch eine den Benutzer/Eigentümer identifizierende Farbkodierung gezeigt, sowie die optional vom Inkubator registrierten Zeitpunkte des Einstellens der Objekte in die Inkubatorkammer.

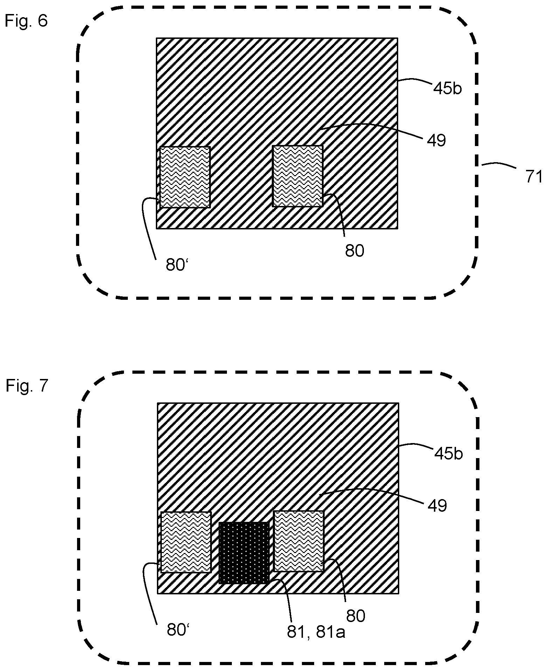

Fig. 6 zeigt einen Lagerbereich, nämlich die Oberseite des Regalblecheinsatzes 45b, aus einer Vogelperspektive bzw. in Aufsicht. Schematisch gezeigt ist zudem der Bilderfassungsausschnitt 71, den die Kamera 70 erfasst. Der Bilderfassungsausschnitt 71 ist der Bereich, der von der Kamera 70 in einem bzw. in jedem Bild erfasst wird, denn die Kamera 70 ändert ihren Blickwinkel und ihre Position hier nicht. Somit zeigt jedes Bild diesen Ausschnitt 71. In den Figuren stellt die untere Kante des Ausschnitts 71 den nahe der Inkubatorkammeröffnung 4 gelegenen Bereich dar. Die Kamera 70 und oder die Beleuchtungseinrichtung 90 mit den beiden Lichtquellen (LEDs) 90' und 90" können aber auch mittels der Transporteinrichtung 95, hier ein motorisiertes Schienensystem, bewegbar bzw. verfahrbar gelagert sein.

Das Bilderfassungssystem 20, 200 ist hier dazu eingerichtet,
- mittels der Beleuchtungseinrichtung 90 den sich zwischen den Innenwänden 72a, 72b erstreckenden Lagerbereich 49 zu beleuchten,
- mittels der Kameraeinrichtung 70 mindestens ein Bild des sich zwischen den Innenwänden 72a, 72b erstreckenden Lagerbereichs 49 zu erfassen, und
- das mindestens eine Bild mittels der Datenverarbeitungseinrichtung 21 in Form von Bilddaten in der Datenspeichereinrichtung 22 zu speichern.

Das Bilderfassungssystem ist zudem dazu eingerichtet,
- mittels der Beleuchtungseinrichtung 90 mindestens zwei auf diesem Lagerbereich 49 angeordnete Objekte 80, 80' zu beleuchten,
- mittels der Kameraeinrichtung 70 ein Bild der mindestens zwei Objekte 80, 80' auf diesem Lagerbereich 49 zu erfassen -also ein Bild auf dem diese mindestens zwei Objekte gezeigt sind-, und
- das Bild der mindestens zwei Objekte 80, 80' und des Lagerbereichs 49 mittels der Datenverarbeitungseinrichtung 21 in Form von Bilddaten in der Datenspeichereinrichtung 22 zu speichern.

Die Datenverarbeitungseinrichtung ist hier optional dazu programmiert,
- mittels Auswertung der Bilddaten das in dem Bild dargestellte erste Objekt 80 und zweite Objekt 80' zu unterscheiden (insbesondere: dem ersten und zweiten Objekt unterschiedliche Identifikationsdaten zuzuordnen; die Objekte zu zählen; die Objektklasse zu erkennen; Individualmerkmale zu analysieren, speichern, erkennen; Objekte bei Bewegung zu verfolgen), insbesondere mittels Bildverarbeitungsalgorithmen die Umrisse des ersten 80 und zweiten Objektes 80' in dem Bild zu erfassen, und
- insbesondere Informationen über das erste 80 und zweite Objekt 80', insbesondere die Umrisse des ersten 80 und zweiten Objektes 80', in Form von Objektdaten in der Datenspeichereinrichtung 22 zu speichern.

Die Beleuchtungseinrichtung 90 ist hier optional betreibbar und die Datenverarbeitungseinrichtung ist hier optional dazu programmiert, dass die Beleuchtungseinrichtung 90 in mindestens zwei verschiedenen Beleuchtungsmodi betrieben wird, und das Bilderfassungssystem 20, 200 ist dazu eingerichtet,
- den Lagerbereich 49 der Inkubatorkammer mittels der Beleuchtungseinrichtung 90
   o zunächst in einem ersten Beleuchtungsmodus, insbesondere mit aktiver LED 90' und inaktiver LED 90", und
   o danach in einem davon verschiedenen zweiten Beleuchtungsmodus, insbesondere mit aktiver LED 90" und inaktiver LED 90', zu beleuchten,
- mindestens ein Bild des Lagerbereichs 49 während der Beleuchtung sowohl mittels des ersten als auch des zweiten Beleuchtungsmodus mittels der Kameraeinrichtung 70 zu erfassen, und
- das mindestens eine Bild in Form von Bilddaten bereitzustellen, die sowohl während des ersten als auch des zweiten Beleuchtungsmodus erfasste kombinierte Bildinformationen enthalten, wobei die Datenverarbeitungseinrichtung dazu programmiert ist, ein Bildauswertungsprogramm auszuführen, das die kombinierten Bildinformationen aus den Bilddaten gewinnt.

Vorzugsweise enthält das mindestens eine Bild des Lagerbereichs 49 mindestens ein erstes Bild des Lagerbereichs 49 und ein davon verschiedenes zweites Bild des Lagerbereichs 49, wobei das erste Bild im ersten Beleuchtungsmodus erfasst wird und das zweite Bild im zweiten Beleuchtungsmodus erfasst wird, und das erste Bild in Form von ersten Bilddaten und das zweite Bild in Form von zweiten Bilddaten bereitgestellt wird, wobei die Datenverarbeitungseinrichtung und das Bildauswertungsprogramm so programmiert sind, dass
- die ersten Bilddaten und die zweiten Bilddaten kombiniert werden, um kombinierte Bilddaten zu erhalten, die insbesondere durch eine Addition und/oder Mittelung von ersten und zweiten Bilddaten entsteht, und
- die kombinierten Informationen aus den kombinierten Bilddaten gewonnen werden.

Die Datenverarbeitungseinrichtung des Bilderfassungssystems 20, 200 ist dazu programmiert, abhängig von der Detektion des geschlossenen Zustands der Außentüre 41 des Inkubators die Bilddaten mittels der Kamera 70 während Beleuchtung zu erfassen und auszuwerten. Mittels Vergleich von zeitlich aufeinanderfolgenden Bildern kann festgestellt werden, ob ein neues Objekt 81 in den Kameraausschnitt 71 gelangt ist.

Fig. 7: Ein Bild, das einen neu im Ausschnitt 71 erschienen Umriss 81a enthält, der dem in den Innenraum eingebrachten Objekt 81 zuordenbar ist, wird als geändertes Bild angesehen. Ausgehend von diesem geänderten Bild werden dem neu erschienenen Umriss 81a Identifikationsdaten zugeordnet, in der Annahme, dass es sich um ein neu in den Inkubator einzustellendes Objekt 81 handelt.

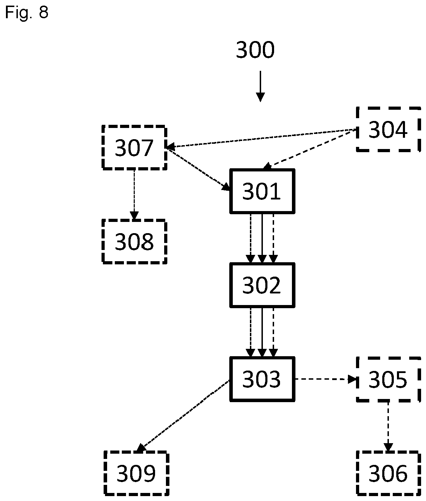

Fig. 8 zeigt den Ablauf des erfindungsgemäßen Verfahrens, das auch schon in der obigen Beschreibung der vorherigen Figuren indirekt beschrieben war.

Das Verfahren 300 dient der Bilderfassung in einem Inkubator, welcher der Inkubation lebender Zellkulturen dient, und der aufweist:
- eine Inkubatorkammer zurAufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt,
- eine Inkubatortüre zum Verschließen der Kammeröffnung,
- ein Bilderfassungssystem, aufweisend
   ∘ eine Beleuchtungseinrichtung,
   ∘ eine Kameraeinrichtung und
   ∘ eine Datenverarbeitungseinrichtung mit einem Datenspeicher,
wobei das Verfahren 300 die programmgesteuerten Schritte aufweist:
- Beleuchten des sich zwischen den Innenwänden erstreckenden Lagerbereichs mittels der Beleuchtungseinrichtung, (301)
- Erfassen mindestens eines Bildes des sich zwischen den Innenwänden erstreckenden Lagerbereichs während der Beleuchtung mittels der Kameraeinrichtung, (302) und
- Speichern des mindestens einen Bildes in Form von Bilddaten in der Datenspeichereinrichtung. (303)

Vorzugsweise auch die Schritte:
- Überwachen, im Zeitverlauf, der Inkubatorkammer 2, 5 mittels mindestens einer Kamera 70 der Kameraeinrichtung 70' des Inkubators, die zur Aufnahme mindestens eines Lagerbereichs 49 im Innenraum der Inkubatorkammer angeordnet ist, in den das mindestens eine Objekt 80; 80'; 81 eingebracht wird; (304)
- Zuordnen von Identifikationsdaten zu dem mindestens einen Objekt 80; 81, das in einem mittels der mindestens einen Kamera 70 aufgenommenen Bild des Lagerbereichs 49 nach dessen Positionieren in dem Lagerbereich erfasst wird; (305)
- Speichern der Position des mindestens einen Objektes 80; 81 im Lagerbereich 49 in Abhängigkeit von den Identifikationsdaten des mindestens einen Objektes als ID-Positionsdaten, in dem Datenspeicher. (306)

Vorzugsweise beinhaltet das Verfahren 300 auch die Schritte:
- Einlesen von einen den Benutzer des Inkubators 1, der das mindestens eine Objekt 80; 81 in die Inkubatorkammer einbrachte, identifizierenden Benutzeridentifikationsdaten mittels einer Benutzeridentifikationseinrichtung (307) und
Speichern der Benutzeridentifikationsdaten in einem Datenspeicher des Inkubators. (308)

Vorzugsweise beinhaltet das Verfahren 300 auch den Schritt:
Speichern der ID-Positionsdaten in Abhängigkeit von den Benutzeridentifikationsdaten als benutzerbezogene ID-Positionsdaten. (309)

## Patentansprüche

1. Inkubator zur Inkubation lebender Zellkulturen, aufweisend
eine Inkubatorkammer zur Aufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt,
eine Inkubatortüre zum Verschließen der Kammeröffnung,
ein Bilderfassungssystem, aufweisend
• eine Beleuchtungseinrichtung,
• mindestens eine Kameraeinrichtung und
• eine Datenverarbeitungseinrichtung mit einer Datenspeichereinrichtung,
wobei das Bilderfassungssystem dazu eingerichtet ist,
• mittels der Beleuchtungseinrichtung den sich zwischen den Innenwänden erstreckenden Lagerbereich zu beleuchten,
• mittels der Kameraeinrichtung mindestens ein Bild des sich zwischen den Innenwänden erstreckenden Lagerbereichs zu erfassen, und
• das mindestens eine Bild mittels der Datenverarbeitungseinrichtung in Form von Bilddaten in der Datenspeichereinrichtung zu speichern.

2. Inkubator gemäß Anspruch 1, wobei das Bilderfassungssystem dazu eingerichtet ist,
• mittels der Beleuchtungseinrichtung mindestens zwei auf diesem Lagerbereich angeordnete Objekte zu beleuchten,
• mittels der Kameraeinrichtung ein Bild der mindestens zwei Objekte auf diesem Lagerbereich zu erfassen, und
• das Bild der mindestens zwei Objekte mittels der Datenverarbeitungseinrichtung in Form von Bilddaten in der Datenspeichereinrichtung zu speichern.

3. Inkubator gemäß Anspruch 2, wobei die Datenverarbeitungseinrichtung dazu programmiert ist,
• mittels Auswertung der Bilddaten das in dem Bild dargestellte erste Objekt und zweite Objekt zu unterscheiden, dem ersten und zweiten Objekt unterschiedliche Identifikationsdaten zuzuordnen; und
• insbesondere Informationen über das erste und zweite Objekt, insbesondere die Bounding Box(es) und/oder Umrisse des ersten und zweiten Objektes, in Form von Objektdaten in der Datenspeichereinrichtung zu speichern.

4. Inkubator gemäß einem der vorangehenden Ansprüche, wobei die Beleuchtungseinrichtung dazu eingerichtet ist, in mindestens zwei verschiedenen Beleuchtungsmodi betrieben zu werden, und das Bilderfassungssystem dazu eingerichtet ist,
• den Lagerbereich der Inkubatorkammer mittels der Beleuchtungseinrichtung
∘ zunächst in einem ersten Beleuchtungsmodus und
∘ danach in einem davon verschiedenen zweiten Beleuchtungsmodus zu beleuchten,
• mindestens ein Bild des Lagerbereichs während der Beleuchtung sowohl mittels des ersten als auch des zweiten Beleuchtungsmodus mittels der Kameraeinrichtung zu erfassen, und
• das mindestens eine Bild in Form von Bilddaten bereitzustellen, die sowohl während des ersten als auch des zweiten Beleuchtungsmodus erfasste kombinierte Bildinformationen enthalten,
wobei die Datenverarbeitungseinrichtung dazu programmiert ist, ein Bildauswertungsprogramm auszuführen, das die kombinierten Bildinformationen aus den Bilddaten gewinnt.

5. Inkubator gemäß Anspruch 4, wobei das mindestens eine Bild des Lagerbereichs mindestens ein erstes Bild des Lagerbereichs und ein davon verschiedenes zweites Bild des Lagerbereichs enthält,
wobei das erste Bild im ersten Beleuchtungsmodus erfasst wird und das zweite Bild im zweiten Beleuchtungsmodus erfasst wird, und das erste Bild in Form von ersten Bilddaten und das zweite Bild in Form von zweiten Bilddaten bereitgestellt wird,
wobei die Datenverarbeitungseinrichtung und das Bildauswertungsprogramm so programmiert sind, dass
• die ersten Bilddaten und die zweiten Bilddaten kombiniert werden, um kombinierte Bilddaten zu erhalten, die insbesondere durch eine Addition und/oder Mittelung von ersten und zweiten Bilddaten entsteht, und
• die kombinierten Informationen aus den kombinierten Bilddaten gewonnen werden.

6. Inkubator gemäß Anspruch 4, wobei das mindestens eine Bild des Lagerbereichs ein mehrfach belichtetes Bild des Lagerbereichs enthält, wobei das Bilderfassungssystem dazu eingerichtet ist,
• das Bild des Lagerbereichs während der Beleuchtung sowohl mittels des ersten als auch des zweiten Beleuchtungsmodus mittels der Kameraeinrichtung zu belichten und zu erfassen, und
• das mehrfach belichtete Bild in Form der Bilddaten bereitzustellen.

7. Inkubator gemäß einem der vorangehenden Ansprüche, wobei das mindestens eine Bild Informationen über im Lagerbereich angeordneten Objekte enthält, insbesondere Informationen, wahlweise,
* über die Positionen der Objekte
* über die Außenkonturen der Objekte,
* über die Fläche der Objekte, gemessen in einer Ebene parallel zu einer planaren Fläche des Lagerbereichs,
* die von den Objekten nicht belegte Fläche des Lagerbereichs, gemessen in einer Ebene parallel zu einer planaren Fläche des Lagerbereichs.

8. Inkubator gemäß einem der vorangehenden Ansprüche, wobei die Beleuchtungseinrichtung mindestens eine erste und eine zweite Lichtquelle aufweist, die im ersten und zweiten Beleuchtungsmodus unterschiedlich betrieben werden,
wobei insbesondere die erste und eine zweite Lichtquelle in einem Abstand oberhalb einer Lagerfläche des Lagerbereichs angeordnet sind,
wobei insbesondere die erste und zweite Lichtquelle in einer Ebene, die parallel zu einer planaren Lagerfläche des Lagerbereichs liegt, versetzt angeordnet sind,
wobei insbesondere der Lagerbereich eine planare Lagerfläche aufweist, wobei die erste Lichtquelle senkrecht oberhalb einer ersten Hälfte der Lagerfläche angeordnet ist und die zweite Lichtquelle senkrecht oberhalb einer zweiten Hälfte der Lagerfläche angeordnet ist,
wobei insbesondere die Beleuchtungseinrichtung ein LED-Leuchtband mit mehreren LED-Lichtquellen aufweist, das in einer Ebene, die parallel zu einer planaren Lagerfläche des Lagerbereichs liegt, angeordnet ist, insbesondere in einem mäandernden Verlauf, einem spiralartigen Verlauf, insbesondere in einem zumindest abschnittsweise linearen Verlauf angeordnet ist,
wobei insbesondere das Bilderfassungssystem eine, insbesondere programmierbare, elektronische Steuereinrichtung aufweist, die so eingerichtet bzw. programmiert ist, dass
∘ während einer Beleuchtungsphase des ersten Beleuchtungsmodus die erste Lichtquelle anders betrieben wird als während einer Beleuchtungsphase des zweiten Beleuchtungsmodus, und/oder
∘ während einer Beleuchtungsphase des ersten Beleuchtungsmodus die zweite Lichtquelle anders betrieben wird als während einer Beleuchtungsphase des zweiten Beleuchtungsmodus,
insbesondere dass
∘ während einer Beleuchtungsphase des ersten Beleuchtungsmodus die erste Lichtquelle aktiv ist und während einer Beleuchtungsphase des zweiten Beleuchtungsmodus weniger aktiv oder inaktiv ist, und/oder
∘ während einer Beleuchtungsphase des ersten Beleuchtungsmodus die zweite Lichtquelle weniger aktiv oder inaktiv ist und während einer Beleuchtungsphase des zweiten Beleuchtungsmodus aktiv ist,
insbesondere dass
∘ während einer Beleuchtungsphase des ersten Beleuchtungsmodus die erste Lichtquelle mit einem anderen Emissionsspektrum betrieben wird als während einer Beleuchtungsphase des zweiten Beleuchtungsmodus, und/oder
∘ während einer Beleuchtungsphase des ersten Beleuchtungsmodus die zweite Lichtquelle mit einem anderen Emissionsspektrum betrieben wird als während einer Beleuchtungsphase des zweiten Beleuchtungsmodus,

9. Inkubator gemäß einem der vorangehenden Ansprüche, wobei
insbesondere die mindestens eine Kamera in einem Abstand senkrecht oberhalb zu einer Lagerfläche des Lagerbereichs angeordnet ist,
insbesondere die mindestens eine Kamera eine Weitwinkeloptik aufweist, insbesondere ein Fischaugenobjektiv aufweist,
insbesondere genau eine Kamera vorgesehen ist, die in einem Abstand senkrecht oberhalb zu einem Zentrum der Lagerfläche des Lagerbereichs angeordnet ist.

10. Inkubator gemäß einem der vorangehenden Ansprüche, wobei
insbesondere das Bilderfassungssystem ein modularer, nämlich vom Benutzer wahlweise einsetzbarer Bestandteil des Inkubators ist,
wobei insbesondere der Inkubator eine Steuereinrichtung und eine Temperiereinrichtung zur Regelung der Temperatur im Innenraum der Inkubatorkammer aufweist, wobei das Bilderfassungssystem eine andere Steuereinrichtung aufweist, die dazu eingerichtet ist, das Bilderfassungssystem zu steuern, insbesondere indem diese andere Steuereinrichtung die Datenverarbeitungseinrichtung des Bilderfassungssystems beinhaltet,
wobei insbesondere der Inkubator eine Steuereinrichtung und eine Temperiereinrichtung zur Regelung der Temperatur im Innenraum der Inkubatorkammer aufweist, wobei insbesondere diese Steuereinrichtung dazu eingerichtet ist, das Bilderfassungssystem zu steuern, insbesondere indem diese Steuereinrichtung die Datenverarbeitungseinrichtung des Bilderfassungssystems beinhaltet.

11. Inkubator gemäß einem der vorangehenden Ansprüche, wobei der Inkubator in Display aufweist und dazu eingerichtet bzw. programmiert ist, auf dem Display vorzugsweise das Bild, und/oder
vorzugsweise aus dem mindestens einen Bild entnommene Bildinformationen, und/oder
vorzugsweise ein die kombinierten Bildinformationen enthaltendes Bild des Lagerbereichs anzuzeigen.

12. Inkubator gemäß einem der vorangehenden Ansprüche, wobei das Bilderfassungssystem ein Objekterfassungssystem ist, indem die Datenverarbeitungseinrichtung dazu programmiert ist, das während der Bilderfassung des mindestens einen Bildes im Lagerbereich angeordnete mindestens eine Objekt mittels des Bildauswertungsprogramms zu erfassen.

13. Inkubator gemäß einem der vorhergehenden Ansprüche, wobei die Datenverarbeitungseinrichtung dazu programmiert ist, durch Bildverarbeitung der Bilddaten mindestens ein in dem Lagerbereich gelagertes Objekt zu erkennen, dem mindestens einen Objekt im Lagerbereich jeweils Identifikationsdaten zuzuordnen und insbesondere die Position des mindestens einen Objektes jeweils als ID-Positionsdaten zu ermitteln und in dem Datenspeicher zu speichern, wobei der Inkubator insbesondere einen Bildschirm aufweist und die Datenverarbeitungseinrichtung dazu programmiert ist, eine graphische Wiedergabe des Lagerbereichs auf dem Bildschirm anzuzeigen, und insbesondere graphisch anzuzeigen, wo das durch die ID-Positionsdaten identifizierte Objekt positioniert ist, bzw. um graphisch anzuzeigen, wo alle im Lagerbereich bzw. Innenraum lokalisierten Objekte angeordnet sind.

14. Inkubator gemäß Anspruch 13, wobei die Datenverarbeitungseinrichtung dazu programmiert ist, den ID-Positionsdaten jeweils Benutzeridentifikationsdaten zuzuordnen und als benutzerbezogene ID-Positionsdaten im Datenspeicher zu speichern, und insbesondere nachfolgend ausgehend von vorgegebenen Benutzeridentifikationsdaten einen Abgleich mit im Datenspeicher gespeicherten benutzerbezogenen ID-Positionsdaten durchzuführen um festzustellen, wo die diesen vorgegebenen Benutzeridentifikationsdaten mittels der benutzerbezogenen ID-Positionsdaten zugeordneten Objekte aktuell im Lagerbereich bzw. im Innenraum positioniert sind und, insbesondere, diese Objekte auf dem Bildschirm graphisch zu markieren.

15. Inkubator gemäß einem der Ansprüche 13 oder 14, wobei die Datenverarbeitungseinrichtung dazu programmiert ist, auf dem Bildschirm freien Lagerplatz anzuzeigen, und/oder aus den ID-Positionsdaten abgeleitete Informationen, insbesondere die Identität des Benutzers, der die Positionsänderung herbeigeführt hat, und / oder statistische Informationen, insbesondere die Häufigkeit und den Zeitpunkt der Positionsänderung eines Objektes und / oder den prozentual verfügbaren freien Lagerplatz.

16. System zur Inkubation lebender Zellkulturen, aufweisend
einen Inkubator zur Inkubation lebender Zellkulturen, der aufweist:
eine Inkubatorkammer zur Aufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt, eine Inkubatortüre zum Verschließen der Kammeröffnung,
ein zum Nachrüsten des Inkubators eingerichtetes bildverarbeitendes Bilderfassungssystem, das eine Datenverarbeitungseinrichtung mit einem Datenspeicher, eine Beleuchtungseinrichtung, und eine Kameraeinrichtung aufweist, die dazu eingerichtet ist, mindestens einen Lagerbereich der Inkubatorkammer zu erfassen,
wobei das Bilderfassungssystem eingerichtet ist, und insbesondere die Datenverarbeitungseinrichtung dazu programmiert ist,
• mittels der Beleuchtungseinrichtung den sich zwischen den Innenwänden erstreckenden Lagerbereich des Inkubators zu beleuchten,
• mittels der Kameraeinrichtung mindestens ein Bild des sich zwischen den Innenwänden erstreckenden Lagerbereich zu erfassen, und
• das mindestens eine Bild mittels der Datenverarbeitungseinrichtung in Form von Bilddaten in der Datenspeichereinrichtung zu speichern.

17. Verfahren zur Bilderfassung in einem Inkubator, welcher der Inkubation lebender Zellkulturen dient, und der aufweist:
• eine Inkubatorkammer zur Aufnahme von Objekten, insbesondere Zellkulturbehältern, die gegenüberliegende Innenwände und eine Kammeröffnung für die Zuführung und die Entnahme der Objekte durch einen Benutzer aufweist, und die mindestens einen Lagerbereich zum Lagern der Objekte aufweist, der sich zwischen den gegenüberliegenden Innenwänden erstreckt,
• eine Inkubatortüre zum Verschließen der Kammeröffnung,
• ein Bilderfassungssystem, aufweisend
∘ eine Beleuchtungseinrichtung,
∘ eine Kameraeinrichtung und
∘ eine Datenverarbeitungseinrichtung mit einem Datenspeicher,
wobei das Verfahren die Schritte aufweist:
• Beleuchten des sich zwischen den Innenwänden erstreckenden Lagerbereichs mittels der Beleuchtungseinrichtung,
• Erfassen mindestens eines Bildes des sich zwischen den Innenwänden erstreckenden Lagerbereichs während der Beleuchtung mittels der Kameraeinrichtung, und
• Speichern des mindestens einen Bildes in Form von Bilddaten in der Datenspeichereinrichtung.

18. Verfahren gemäß Anspruch 17, aufweisend die Schritte:
Erkennen mindestens eines in dem Lagerbereich gelagerten Objektes mittels Bildverarbeitung;
Zuordnen von Identifikationsdaten zu jedem Objekt im Lagerbereich;
Ermitteln der Position des mindestens einen Objektes im Lagerbereich bzw. Innenraum als ID-Positionsdaten Speichern der in dem Datenspeicher zu speichern;
Anzeigen einer graphischen Wiedergabe des Lagerbereichs auf einem Bildschirm des Inkubators anzuzeigen, und insbesondere Anzeigen der Information, wo das durch die ID-Positionsdaten identifizierte Objekt positioniert ist, bzw. wo alle im Lagerbereich bzw. Innenraum lokalisierten Objekte angeordnet sind;
insbesondere Visualisierung der gespeicherten ID-Positionsdaten und/oder der benutzerbezogenen ID-Positionsdaten auf dem Bildschirm, insbesondere durch eine graphische Wiedergabe des Innenraums der Inkubatorkammer auf dem Bildschirm, und insbesondere das graphische Anzeigen der Information, wo das durch die ID-Positionsdaten identifizierte Objekt positioniert ist, bzw., wo alle im Innenraum lokalisierten Objekte angeordnet sind, oder Anzeigen des freien Lagerplatzes im Innenraum des Inkubators auf dem Bildschirm.
